# FASCICULE DE BREVET EUROPEEN

(11) **EP 2 872 647 B1**
(45) Date de publication et mention de la délivrance du brevet: **14.10.2020**
(21) Numéro de dépôt: 13736921.1
(22) Date de dépôt: 15.07.2013
(51) Int. Cl.: C12Q 1/6888, C12Q 1/6876, G01N 33/92, G01N 33/50, G01N 33/68

(54) **PROCÉDÉ D'IDENTIFICATION DE MARQUEURS MOLÉCULAIRES DE LA PEAU D'ENFANT**
VERFAHREN ZUR IDENTIFIZIERUNG MOLEKULARE MARKER VON KINDERHAUT
METHOD FOR IDENTIFYING MOLECULAR MARKERS OF CHILDREN'S SKIN

(30) Priorité: 13.07.2012 FR 1256766
(43) Date de publication de la demande: 20.05.2015
(73) Titulaire: Laboratoires Expanscience, 92048 Paris La Défense Cedex (FR)
(72) Inventeur: MSIKA, Philippe, 78000 Versailles (FR); BAUDOUIN, Caroline, 78120 Rambouillet (FR)
(74) Mandataire: Regimbeau
(86) Numéro de dépôt international: PCT/EP2013/064926
(87) Numéro de publication internationale: WO 2014/009566

(56) Documents cités:
- WO-A1-2010/053254
- WO-A1-2011/069913
- WO-A2-01/12851
- FR-A1- 2 944 437
- FR-A1- 2 965 357
- US-A1- 2003 068 663
- LAN WEI ET AL: "Screening of differential expression genes of human skin epidermal stem cells at different development stages by cDNA microarray technique", ZHONGHUA SHAOSHANG ZAZHI - CHINESE JOURNAL OF BURNS, ZHONGHUA YIXUEHUI, CN, vol. 27, 1 février 2011 (2011-02-01), pages 26-31, XP009167452, ISSN: 1009-2587
- MICHEL MARTINE ET AL: "Keratin 19 as a biochemical marker of skin stem cells in vivo and in vitro: Keratin 19 expressing cells are differentially localized in function of anatomic sites, and their number varies with donor age and culture stage", JOURNAL OF CELL SCIENCE, CAMBRIDGE UNIVERSITY PRESS, LONDON, GB, vol. 109, no. 5, 1 janvier 1996 (1996-01-01), pages 1017-1028, XP002216781, ISSN: 0021-9533
- GILCHREST B A GARMYN M YAAR M: "Aging and photoaging affect gene expression in cultured human keratinocytes", ARCHIVES OF DERMATOLOGY, AMERICAN MEDICAL ASSOCIATION, US, vol. 130, no. 1, 1 janvier 1994 (1994-01-01), pages 82-86, XP009015901, ISSN: 0003-987X, DOI: 10.1001/ARCHDERM.130.1.82
- J.W. FLUHR ET AL: "Infant epidermal skin physiology: adaptation after birth", BRITISH JOURNAL OF DERMATOLOGY, vol. 166, no. 3, 1 mars 2012 (2012-03-01), pages 483-490, XP055054059, ISSN: 0007-0963, DOI: 10.1111/j.1365-2133.2011.10659.x
- P. AGACHE ET AL: "Sebum levels during the first year of life*", BRITISH JOURNAL OF DERMATOLOGY, vol. 103, no. 6, 1 décembre 1980 (1980-12-01), pages 643-650, XP055055096, ISSN: 0007-0963, DOI: 10.1111/j.1365-2133.1980.tb01686.x
- KATSUKO KIKUCHI ET AL: "Impairment of Skin Barrier Function is not Inherent in Atopic Dermatitis Patients: A Prospective Study Conducted in Newborns", PEDIATRIC DERMATOLOGY, vol. 23, no. 2, 1 mars 2006 (2006-03-01), pages 109-113, XP055055095, ISSN: 0736-8046, DOI: 10.1111/j.1525-1470.2006.00191.x
- PERKINS M A ET AL: "a noninvasive method to assess skin irritation and compromised skin conditions using simple tape absorption of molecular markers of inflammation", SKIN RESEARCH AND TECHNOLOGY, MUNKSGAARD, COPENHAGEN, DK, vol. 7, 1 novembre 2001 (2001-11-01), pages 227-237, XP002260517, ISSN: 0909-752X, DOI: 10.1034/J.1600-0846.2001.70405.X
- COQUETTE A ET AL: "Analysis of interleukin-1alpha (IL-1alpha) and interleukin-8 (IL-8) expression and release in in vitro reconstructed human epidermis for the prediction of in vivo skin irritation and/or sensitization", TOXICOLOGY IN VITRO, ELSEVIER SCIENCE, GB, vol. 17, no. 3, 1 juin 2003 (2003-06-01), pages 311-321, XP008108486, ISSN: 0887-2333
- THOMAS WELSS ET AL: "In vitro skin irritation: facts and future. State of the art review of mechanisms and models", TOXICOLOGY IN VITRO, vol. 18, no. 3, 1 juin 2004 (2004-06-01), pages 231-243, XP055056718, ISSN: 0887-2333, DOI: 10.1016/j.tiv.2003.09.009
- Anonymous: "Quality controls-Biological safety controls before tissue engineering", , 16 décembre 2011 (2011-12-16), XP002713382, Extrait de l'Internet: URL:http://web.archive.org/web/20111216181 524/http://www.skinethic.com/pageLibre0001 01ac.asp
- Sang Woong Youn ET AL: "Cellular senescence induced loss of stem cell proportion in the skin in vitro", Journal of Dermatological Science, vol. 35, no. 2, 1 August 2004 (2004-08-01) , pages 113-123, XP055562325, AMSTERDAM, NL ISSN: 0923-1811, DOI: 10.1016/j.jdermsci.2004.04.002

## Description

### Introduction

La peau est un ensemble de cellules et de macromolécules regroupées sous forme d'un tissu résistant et souple, recouvrant la totalité du corps. La peau a pour fonction principale d'établir une barrière de protection contre les atteintes de l'environnement tout en permettant certains échanges entre le milieu intérieur et le milieu extérieur. Elle est le siège de nombreux processus métaboliques qui sont modulés par les conditions physiologiques de l'organisme et les conditions de l'environnement. La peau est formée de deux couches jointes : l'épiderme et le derme auxquelles on peut associer les tissus sous-cutanés.

L'épiderme dont le principal rôle est la protection du corps constitue la couche la plus superficielle de la peau et assure l'imperméabilité de la peau et sa résistance. On peut identifier dans celui-ci 4 couches cellulaires distinctes, une couche basale (*stratum basalis*), une couche épineuse (*stratum spinosum*), une couche granuleuse (*stratum granulosum*), et une couche cornée (*stratum corneum*). Si différents types cellulaires coexistent dans l'épiderme, les kératinocytes sont largement majoritaires (90 %). Leur activité caractéristique est la synthèse des kératines qui représentent 95 % des protéines totales de l'épiderme. Les kératines, protéines fibreuses et insolubles dans l'eau, entrent dans la constitution de la couche cornée de l'épiderme qui protège la peau contre les agressions extérieures (chaleur, froid, déshydratation).

L'épiderme est relié au derme par une zone appelée jonction dermo-épidermique ou membrane basale épidermique. Cette structure assure l'adhérence du derme à l'épiderme et joue le rôle de support mécanique responsable en partie de la tonicité de la peau. Elle est élaborée à la fois par les kératinocytes basaux et par les fibroblastes dermiques et elle contient un taux important de collagène de type IV qui fait partie des plaques d'ancrage reliant la membrane basale à des plaques d'ancrage présentes dans le derme papillaire.

Le derme, la couche interne de la peau, est un tissu conjonctif fibro-élastique composé de cellules (les fibroblastes) dispersées dans un milieu complexe appelé matrice extracellulaire. Cette matrice est constituée de fibres de collagène et d'élastine, de glycoprotéines (fibronectine et laminine) et de protéoglycanes (protéine centrale + glycosaminoglycanes ou GAGs). La nature et la quantité de ces composants régissent les propriétés mécaniques de la peau et sont à l'origine des modifications physiopathologiques les plus visibles du relief cutané observées au cours du vieillissement.

L'hypoderme est le compartiment le plus profond et le plus épais de la peau. Il s'invagine dans le derme et est rattaché au derme sous-jacent par des fibres de collagène et d'élastine. Il est essentiellement constitué d'un type de cellules spécialisées dans l'accumulation et le stockage des graisses, les adipocytes. Les adipocytes constituant l'hypoderme sont des cellules regroupées en lobules séparés par du tissu conjonctif. Il sert de réserve énergétique, mais aussi de protection thermique et mécanique.

L'adaptation à la vie extra-utérine est un processus qui commence dès la naissance et se poursuit tout au long de la première année de la vie. Les premiers mois de la vie postnatale constituent une période de réorganisation fonctionnelle de la peau qui permet une adaptation physiologique à l'environnement extra-utérin.

Par exemple, l'acidification de la peau augmente après la naissance pour atteindre à environ un mois des valeurs similaires à celles observées chez les adultes. En revanche, la sécrétion de sébum est importante à la naissance et diminue dès la fin de la première semaine pour rester à un niveau inférieur à celui mesuré chez l'adulte et ce jusqu'à la puberté (Fluhr et al., Exp Dermatol., 19(6) : 483-492, 2010).

Une étude clinique récente associant des mesures biométrologiques classiques à la technique de la spectrométrie Raman (Fluhr et al., Br J Dermatol, 166(3) : 483-90, 2012) a montré que le taux d'hydratation et le contenu en eau de la couche cornée sont plus faibles à la naissance (nouveau-nés de 1 à 15 jours), alors que le taux de facteur hydratant NMF (Natural Moisturing Factor) est maximal. Ce taux diminue ensuite alors que l'hydratation se stabilise. Les auteurs font l'hypothèse que l'acidification moindre et l'hydratation plus faible de la peau pourraient activer des mécanismes régulateurs de compensation (avec notamment une production importante de NMF), permettant l'adaptation du nouveau-né à son nouvel environnement. Dans la même étude, il a été montré que le taux de NMF chez les nourrissons de 6 mois se trouve inférieur à celui observé dans le groupe d'adultes. Ce moindre taux de NMF, démontré par un autre groupe (Nikolovski et al., J Invest Dermatol, 128 : 1728-36, 2008) suggère que la peau des nourrissons présente une certaine immaturité sur sa capacité à capter l'eau et à réguler les mécanismes qui y sont liés, ce qui aurait un impact sur la qualité et la compétence de la fonction barrière.

Une maturation incomplète de la peau peut avoir des conséquences cliniques importantes. Il est connu par exemple que le développement des pathogènes microbiens est favorisé par des valeurs de pH élevées (Korting et al., Acta Derm Venereol., 70(5) : 429-431, 1990). Il est donc important de ne pas utiliser de détergents qui diminueraient encore l'acidification de la peau dans les premiers mois de la vie.

La compréhension des processus complexes mis en oeuvre au cours des premières étapes de la vie postnatale permettrait donc de pouvoir identifier des agents actifs ou des combinaisons d'agents actifs ayant une tolérance et une efficacité particulièrement adaptée à chaque classe d'âge. Cependant, nous ne disposons pas de marqueurs biologiques et moléculaires de l'évolution de la peau dans les premiers mois, voire les premières années de la vie postnatale

il y a donc un besoin dans le domaine pour l'identification de marqueurs qui soient spécifiques de l'évolution de la peau, depuis la naissance jusqu'à l'âge adulte.

### Légendes des figures

Figure 1 : Évolution de l'expression des gènes de la fonction barrière CLDN1, IVL, KRT1, dans les épidermes reconstruits (A) et dans les kératinocytes (B) en fonction de l'âge. Le niveau d'expression à 1 mois a été fixé arbitrairement à 100 %.
Figure 2 : Évolution de l'expression du gène de réponse au stress GP3X (A) et du gène de l'immunité innée DEFB1 (B) dans les kératinocytes en fonction de l'âge. Le niveau d'expression à 1 mois a été fixé arbitrairement à 100 %.
Figure 3 : Évolution de l'expression du gène de l'inflammation MGST1 dans les kératinocytes en fonction de l'âge. Le niveau d'expression à 1 mois a été fixé arbitrairement à 100 %.
Figure 4: Évolution de l'expression des gènes de cellules souches : FN1, NID1, NOTCH1, KRT19, IGTB1P1, ITGA6 et ITGB4 dans les kératinocytes en fonction de l'âge. Le niveau d'expression à 1 mois a été fixé arbitrairement à 100 %.
Figure 5 : Evaluation par analyse histologique de la tolérance des produits bébés à base de perséose d'avocats sur des épidermes reconstruits provenant de donneurs âgés de 1 mois. Les tissus inclus en paraffine ont été colorés à l'hématoxyline et à l'éosine.

### Description de l'invention

L'invention est décrite dans les revendications jointes. La présente description a pour but de fournir des marqueurs biologiques permettant de caractériser une peau d'enfant, depuis la naissance jusqu'à la puberté.

Le procédé de la description permet ainsi d'identifier des marqueurs moléculaires exprimés de façon différentielle dans la peau d'enfant, c'est-à-dire des marqueurs dont l'expression est différente dans la peau d'enfant et la peau d'adulte. Ledit procédé permet donc de caractériser la peau au niveau moléculaire depuis la naissance et de suivre son évolution au cours du temps.

Par « enfant », on entend ici un individu dont l'âge est inférieur à 16 ans. Sont ainsi compris dans la catégorie des enfants, les nouveau-nés, dont l'âge est compris entre 0 et 1 mois, les nourrissons, qui ont entre 1 mois et 2 ans, et les enfants proprement dits, qui sont âgés d'au moins 2 ans. Un « nouveau-né », comme on l'entend ici, peut aussi bien être né à terme qu'être prématuré.

Pour lever toute ambiguïté, le terme « enfant » utilisé ici sans autre précision doit être entendu dans son acception la plus générale, c'est-à-dire comme se référant à une personne de moins de 16 ans. Un « adulte » est ici une personne qui n'est pas un enfant, autrement dit une personne âgée de plus de 16 ans.

Préférablement, la méthode peut être utilisée quelle que soit l'origine ethnique ou géographique de la peau, ou encore le phototype de celle-ci. Elle peut ainsi être d'origine caucasienne, africaine, asiatique, sud-américaine, mélanésienne ou autre ; elle peut encore présenter un phototype I, II, III, IV, V ou VI, sans que cela ne l'affecte. Celle-ci a en effet pour objet l'identification de marqueurs biologiques caractérisant n'importe quel type de peau et ne dépendant que de l'âge du donneur.

Par ailleurs, l'homme du métier réalisera sans peine que l'enseignement de la présente description peut aussi s'appliquer aux muqueuses. Celles-ci sont de minces couches de tissu épithélial et de tissu conjonctif sous-jacent, qui sont, tout comme la peau, à l'interface entre les milieux intérieurs et extérieurs. D'ailleurs certaines de ces muqueuses sont contigües avec la peau.

Il est ici décrit un procédé d'identification d'au moins un marqueur biologique caractérisant une peau d'enfant de moins de 16 ans, ledit procédé comprenant les étapes suivantes :
a) obtenir un échantillon de cellules cutanées (A), ledit échantillon provenant d'un donneur ayant un âge inférieur à 16 ans,
b) mesurer le niveau d'expression d'un marqueur biologique candidat dans l'échantillon de l'étape a), et
c) déterminer si le marqueur candidat est un marqueur biologique caractérisant une peau d'enfant de moins de 16 ans.

L'invention concerne en particulier un procédé tel que revendiqué dans la revendication 1.

Un « marqueur biologique » est ici défini par au moins une différence entre le métabolisme des cellules d'enfants de moins de 16 ans et le métabolisme des cellules d'adultes. Avantageusement, le marqueur biologique est défini par au moins une différence entre le métabolisme des cellules de nouveau-né, de nourrisson et/ou d'enfants âgés de 2 à 16 ans, et le métabolisme des cellules d'adulte.

Par « marqueur biologique caractérisant une peau d'enfant », on entend ici, un marqueur qui est spécifiquement plus exprimé ou moins exprimé dans la peau d'enfant de moins de 16 ans. Autrement dit, un marqueur biologique caractérisant une peau d'enfant est un marqueur dont l'expression varie avec l'âge et qui est exprimé de façon différentielle entre la peau d'enfant et la peau d'adulte.

Comme indiqué plus haut, la peau évolue fonctionnellement et structurellement dans la période après la naissance. En particulier, des variations de différents paramètres tels que la perte d'eau transépidermale (TEWL), l'hydratation du stratum corneum ou le pH de la surface de la peau sont observés non seulement de façon générale entre 0 et 16 ans, mais aussi, de façon plus particulière, à l'intérieur de cet intervalle d'âge. Ainsi ces paramètres varient entre les nouveau-nés, les nourrissons et les enfants entre 2 et 16 ans (Fluhr et al., Exp Dermatol., 19(6) : 483-492, 2010). En particulier, des variations spécifiques de ces paramètres peuvent être observées à l'intérieur de chacune de ces tranches d'âge.

Le terme de « marqueur biologique caractérisant une peau d'enfant » tel qu'utilisé ici recouvre donc aussi tout marqueur biologique qui est plus exprimé ou qui est moins exprimé chez le nouveau-né et/ou chez le nourrisson et/ou chez l'enfant entre 2 et 16 ans, que chez l'adulte. Un marqueur caractérisant une peau d'enfant peut donc être exprimé de façon plus importante ou moins importante que chez l'adulte dans l'une ou plusieurs de ces trois catégories, voire dans l'ensemble de la catégorie des enfants de moins 16 ans.

Selon un mode de réalisation plus préféré, le donneur de l'échantillon (A) est plus particulièrement un donneur ayant un âge compris entre 0 et 1 mois, entre 1 mois et 2 ans ou entre 2 ans et 16 ans. Autrement dit, selon ce mode de réalisation, le donneur de l'échantillon (A) est choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans.

Dans le procédé de la description, il est possible de déterminer si un marqueur biologique candidat est un marqueur spécifique d'une peau d'enfant en comparant le niveau d'expression du marqueur candidat dans l'échantillon de cellules cutanées (A) et dans un échantillon de cellules cutanées témoin (B).

Il est ici décrit un procédé d'identification d'au moins un marqueur biologique caractérisant une peau d'enfant, ledit procédé comprenant les étapes suivantes :
a) obtenir au moins un échantillon de cellules cutanées (A), ledit échantillon provenant d'un donneur ayant un âge inférieur à 16 ans,
b) obtenir au moins un échantillon témoin (B) de cellules cutanées,
c) mesurer le niveau d'expression d'un marqueur biologique candidat dans l'échantillon de l'étape a),
d) mesurer le niveau d'expression dudit marqueur biologique candidat dans l'échantillon de l'étape b),
e) calculer le rapport entre le niveau d'expression de l'étape a) et le niveau d'expression de l'étape b), et
f) déterminer si le marqueur candidat est un marqueur biologique caractérisant une peau de nouveau-né ou de nourrisson.

De préférence, le procédé ici décrit est un procédé d'identification d'au moins un marqueur biologique caractérisant une peau d'enfant, ledit procédé comprenant les étapes suivantes :
a) obtenir au moins un échantillon de cellules cutanées (A), ledit échantillon provenant d'un donneur choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans ;
b) obtenir au moins un échantillon témoin (B) de cellules cutanées ;
c) mesurer le niveau d'expression d'un marqueur biologique candidat dans l'échantillon de l'étape a) ;
d) mesurer le niveau d'expression dudit marqueur biologique candidat dans l'échantillon de l'étape b) ;
e) calculer le rapport entre le niveau d'expression de l'étape a) et le niveau d'expression de l'étape b) ; et
f) déterminer si le marqueur candidat est un marqueur biologique caractérisant une peau d'enfant.

L'échantillon témoin (B) selon l'invention et la description est un échantillon de cellules cutanées provenant d'un donneur dont l'âge est connu. Par exemple, l'échantillon témoin (B) peut provenir d'un enfant ou d'un adulte. Plus précisément, l'échantillon témoin (B) peut provenir d'un donneur ayant moins de 16 ans ou ayant plus de 16 ans. Avantageusement, l'échantillon témoin (B) du procédé ici décrit provient d'un donneur dont l'âge est compris entre 0 et 1 mois, entre 1 mois et 2 ans ou entre 2 ans et 16 ans. Il est ainsi possible de préciser si le marqueur biologique caractérisant une peau d'enfant est un marqueur caractérisant une peau de nouveau-né de moins de 1 mois, une peau de nourrisson ayant entre 1 mois et 2 ans, ou une peau d'enfant d'âge compris entre 2 et 16 ans.

Il est clair que le rapport de l'étape e) du procédé est dépendant de la nature de l'échantillon témoin (B). On comprendra aisément que si le donneur de l'échantillon témoin (B) provient de la même classe d'âge que le donneur de l'échantillon (A), ledit rapport de l'étape e) sera égal à 1 environ ; en revanche, si les donneurs des échantillons A et B proviennent de deux classes d'âge distinctes, alors le rapport de l'étape e) sera différent de 1. Ainsi, si l'échantillon (B) provient d'un adulte, un rapport entre les niveaux d'expression du marqueur candidat dans l'échantillon (A) et l'échantillon (B) supérieur à 1 indique que ledit marqueur est spécifiquement plus exprimé dans la peau d'enfant. De même, toujours dans le cas où l'échantillon (B) provient d'un adulte, un rapport entre les niveaux d'expression du marqueur candidat dans l'échantillon (A) et l'échantillon (B) inférieur à 1 indique que ledit marqueur est spécifiquement moins exprimé dans la peau d'enfant.

De préférence, le niveau d'expression du marqueur candidat est mesuré dans au moins deux échantillons de cellules cutanées, (A) et (A'), chacun desdits échantillons provenant d'un donneur ayant un âge inférieur à 16 ans. Plus préférentiellement, chacun desdits échantillons provient d'un donneur choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans. Avantageusement, (A) et (A') appartiennent à deux classes d'âge différentes. De manière plus préférée, l'échantillon témoin (B) provient d'un donneur adulte.

II est ainsi possible de tirer des conclusions plus précises et plus fiables concernant le marqueur candidat.

Par « échantillon cutané », on entend ici tout échantillon contenant des cellules de la peau. Les échantillons cutanés comprennent donc aussi bien les explants de peau frais obtenus directement du patient, que les cultures de cellules cutanées en suspension, les cultures de cellules cutanées en monocouche, les cultures de cellules cutanées en bicouche et les modèles tissulaires, dont les cultures de peaux reconstruites et les cultures de muqueuses reconstruites. Comme il est souvent difficile de travailler sur des explants frais, il est particulièrement avantageux d'utiliser des cultures de cellules cutanées. Avantageusement, les cellules cutanées comprennent des cellules normales, saines ou pathologiques, ou des cellules issues de lignées. Par exemple, les cellules cutanées mises en culture peuvent être des cellules obtenues à partir d'explant de tissu cutané. Par « explant » ou « explant de peau », on entend ici un prélèvement de cellules ou de tissu cutané, lequel peut être réalisé dans un but chirurgical ou pour effectuer des analyses.

En particulier, un explant peut être obtenu lors d'une exérèse chirurgicale. Par « exérèse », on entend ici une intervention chirurgicale consistant à découper (exciser) une partie plus ou moins large ou profonde de la peau pour en traiter une anomalie ou une excroissance. On procède à une exérèse soit pour retirer une tumeur cancéreuse ou suspecte de l'être, soit pour traiter une anomalie bénigne de la peau qui est gênante, que ce soit pour des raisons fonctionnelles ou esthétiques. Une exérèse inclut par exemple les échantillons de peau obtenus après chirurgie plastique (plastie mammaire, abdominale, lifting, prélèvement préputial, otoplastie, c'est-à-dire recollement d'oreille, syndactylie ou doigt surnuméraire, etc.).

Un explant peut aussi être obtenu par biopsie. Par « biopsie », on entend ici un prélèvement de cellules ou tissu cutané réalisé à des fins d'analyse. Plusieurs types de procédures de biopsies sont connus et pratiqués dans le domaine. Les types les plus communs comprennent (1) la biopsie incisionnelle, dans laquelle seul un échantillon du tissu est prélevé ; (2) la biopsie excisionnelle (ou biopsie chirurgicale) qui consiste en l'ablation totale d'une masse tumorale, réalisant ainsi un geste thérapeutique et diagnostique, et (3) la biopsie à l'aiguille, dans laquelle un échantillon de tissu est prélevé avec une aiguille, celle-ci pouvant être large ou fine. D'autres types de biopsie existent, comme par exemple le frottis ou le curettage, et sont aussi englobés ici.

Alternativement, lesdites cellules cutanées peuvent être obtenues par différentiation de cellules souches (Guenou et al., Lancet, 374(9703) : 1745-1753, 2009 ; Nissan et al., Proc. Natl. Acad. Sci., 108(36): 14861-14866, 2011 ; Kraehenbuehl et al., Nature Methods, 8 : 731-736, 2011).

Les cellules cutanées, qu'elles proviennent d'une biopsie ou qu'elles soient obtenues par différentiation de cellules souches, comprennent au moins un type de cellules habituellement présentes dans l'hypoderme, le derme et/ou l'épiderme. Ces cellules comprennent ainsi, entre autres, des kératinocytes, des mélanocytes, des fibroblastes, des adipocytes, des cellules endothéliales, des mastocytes, des cellules de Langerhans et/ou des cellules de Merkel. De façon préférentielle, les cellules cutanées selon l'invention et la description comprennent au moins des kératinocytes et/ou des fibroblastes. De façon plus préférentielle, les cellules cutanées comprennent des kératinocytes et/ou des fibroblastes.

De nombreuses méthodes de culture de cellules cutanées sont connues de l'homme du métier. N'importe laquelle de ces méthodes peut être utilisée pour cultiver les cellules cutanées. Avantageusement, les cellules cutanées sont cultivées et/ou conservées dans des conditions maintenant, au moins partiellement, un métabolisme cellulaire et/ou des fonctions cellulaires. La culture de cellules cutanées comprend donc aussi bien les cultures de cellules cutanées en suspension, les cultures de cellules cutanées en monocouche ou les cultures de cellules cutanées en bicouche que des modèles tissulaires, dont les cultures de peaux reconstruites et les cultures de muqueuses reconstruites. De facon préférentielle, culture de cellules cutanées comprend au moins une culture de peau reconstruite.

Par exemple, les cultures de cellules cutanées en suspension sont pratiquées en routine dans un très grand nombre de laboratoires et ce, depuis plusieurs dizaines d'années. De même, les cultures de cellules cutanées en monocouche ou en bicouche sont connues et utilisées depuis très longtemps.

Par ailleurs, de nombreux modèles tissulaires, dont en particulier des modèles de peaux reconstruites et des modèles de muqueuses reconstruites (Rosdy et al., In Vitro Toxicol., 10(1) : 39-47, 1997 ; Ponec et al., J Invest Dermatol., 109(3) : 348-355, 1997 ; Ponec et al., Int J Pharm., 203(1-2) : 211-225, 2000; Schmalz et al., Eur J Oral Sci., 108(5) : 442-448, 2000 ; Black et al., Tissue Eng, 11(5-6) : 723-733, 2005 ; Dongari-Batgtzoglou et Kashleva, Nat Protoc, 1(4) : 2012-2018, 2006 ; Bechtoille et al., Tissue Eng, 13(11) : 2667-2679, 2007 ; Vrana et al., Invest Ophthalmol Vis Sci, 49(12) : 5325-5331, 2008 ; Kinicoglu et al., Biomaterials, 30(32) : 6418-6425, 2009 ; Auxenfans et al., Eur J Dermatol, 19(2): 107-113, 2009 ; Kinicoglu et al., Biomaterials, 32(25): 5756-5764, 2011 ; Costin et al., Altern Lab Anim, 39(4) : 317-337, 2011 ; Auxenfans et al., J Tissue Eng Regen Med, 6(7) : 512-518, 2012 ; Lequeux et al., Skin Pharmacol Physiol, 25(1) : 47-55, 2012; EP 29 678 ; EP 285 471 ; EP 789 074 ; EP 1 451 302 B1 ; EP 1 878 790 B1 ; EP 1 974 718 ; US 2007/0148,771 ; US 2010/0,099,576 ; WO 02/070729 ; WO 2006/063864 ; WO 2006/0,63865 ; WO 2007/064305) sont à la disposition de l'homme du métier.

Avantageusement, le modèle tissulaire comprend les modèles de peau reconstruite et les modèles de muqueuse reconstruite. Préférablement, le modèle de peau reconstruite est sélectionné dans le groupe comprenant les modèles de derme, contenant principalement des cellules stromales, et plus particulièrement encore des fibroblastes, les modèles d'épiderme constitué principalement de kératinocytes, les modèles d'hypoderme, les modèles de peau comprenant un derme et un épiderme, et les modèles de peau comprenant un derme, un épiderme et un hypoderme. Les modèles comprenant au moins un derme, forment des tissus de type conjonctifs, tandis que les modèles comprenant au moins un épiderme forment des épithélia stratifiés comprenant les couches caractéristiques du tissu considéré. Par exemple, on peut identifier dans les modèles d'épiderme une couche basale (*stratum basalis*), une couche épineuse (*stratum spinosum*), une couche granuleuse (*stratum granulosum*), et une couche cornée (*stratum corneum*). D'autre part, de façon préférée, le modèle de muqueuse reconstruite est un modèle de muqueuse de la bouche, de la gencive, du vagin ou de la cornée.

Avantageusement, ledit modèle est un modèle tissulaire de type conjonctif de matrice de derme comprenant un support matriciel de préférence choisi parmi :
- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable, une membrane ou un film d'acide polyglycolique.

Dans ce groupe on trouve par exemple les modèles dermiques Skin^{2TM} modèle ZK1100 et Dermagraft® et Transcyte® (Advanced Tissue Sciences) ;
- un plastique traité culture cellulaire (formation d'un feuillet dermique : Michel et al., In Vitro Cell. Dev Biol.-Animal, 35 : 318-326, 1999) ;
- un gel ou une membrane à base d'acide hyaluronique (Hyalograft® 3D - Fidia Advanced Biopolymers) et/ou de collagène (comme par exemple un derme équivalent ou des lattices de collagène) et/ou de fibronectine et/ou de fibrine ; dans ce groupe on trouve par exemple le modèle dermique Vitrix® (Organogenesis) ;
- une matrice poreuse, surfacée ou non surfacée (par exemple un derme équivalent), réalisée à partir de collagène pouvant contenir un ou plusieurs glycosaminoglycanes et/ou éventuellement du chitosane (EP0296078A1, WO 01/911821 et WO 01/92322).

Dans ce groupe on trouve par exemple le modèle dermique Mimederm® (Coletica).

Ces supports matriciels comprennent des cellules stromales, en particulier des fibroblastes.

Avantageusement, ledit modèle de peau est un modèle d'épiderme comprenant un support matriciel de préférence choisi parmi :
- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, de polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable ;
dans ce groupe on trouve les modèles Epiderme reconstruits (Skinethic®) ainsi que le modèle EpiDerm®, (Mattek Corporation) ;
- un film ou une membrane à base d'acide hyaluronique et/ou de collagène et/ou de fibronectine et/ou de fibrine.

Dans ce groupe on peut citer particulièrement les modèles : Laserskin® (Fidia Advanced Biopolymers), Episkin ® (L'Oréal).

Ces modèles peuvent être ensemencés par des fibroblastes dans la partie dermique.

Ces modèles, dans lesquels peuvent être intégrés ou non des fibroblastes, servent de support pour l'ensemencement des kératinocytes et la reconstitution de l'épiderme. Avantageusement, sont introduites, outre les kératinocytes, des cellules pigmentaires, des cellules immunocompétentes, des cellules nerveuses ; de préférence les cellules immunocompétentes sont des cellules de Langerhans.

Avantageusement, ledit modèle tissulaire est un modèle tissulaire de peau ou de muqueuse reconstruite comprenant un support matriciel dermique ou de chorion de préférence choisi parmi :
- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, de polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable, ledit support inerte contenant ou non des cellules stromales, en particulier des fibroblastes,
- un gel à base de collagène et/ou acide hyaluronique et/ou fibronectine, et/ou de fibrine comprenant des cellules stromales en particulier des fibroblastes,
- une matrice poreuse, surfacée ou non surfacée, réalisée à partir de collagène pouvant contenir un ou plusieurs glycosaminoglycanes et/ou éventuellement du chitosane, ces matrices poreuses intégrant des cellules stromales, en particulier des fibroblastes,
- un derme desépidermisé ou derme mort, humain ou animal.

Dans ce groupe, on peut citer particulièrement les modèles Mimeskin (Coletica), EpidermFT™, EpiAirway™, EpiOccular™ EpiOral™, EpiGingival™, EpiVaginal™ (MatTek corporation), Human Corneal Epithelium (HCE), Human Oral Epithelium (HOE), Human Gingival Epithelium (HGE), Human Vaginal Epithelium (HVE) (Skinethic®), Phenion® Full Thickness Skin Model (Phenion) Apligraf® (Organogenesis), ATS-2000 (CellSystems® Biotechnologie Vertrieb) ainsi que Skin 2TM (ZK1200-1300-2000 Advanced Tissue Science).

Il existe par ailleurs des modèles dédiés à la thérapie tissulaire qui peuvent également être utilisés dans le cadre du présent procédé. On peut citer les modèles Epidex (Modex Thérapeutiques), Epibase® (Laboratoire Genévrier), Epicell™ (Genzyme), Autoderm™ et Transderm™ (Innogenetics).

Le support matriciel est ensuite ensemencé par des kératinocytes pour reconstruire l'épiderme et obtenir finalement une peau reconstruite.

Avantageusement, le modèle de peau utilisé comprend un modèle dans lequel a été incorporé au moins un type cellulaire complémentaire, comme des cellules endothéliales (CE) et/ou des cellules immunitaires telles que lymphocytes, macrophages, mastocytes, cellules dendritiques et/ou des cellules adipeuses et/ou des annexes cutanées, comme des poils, des cheveux, des glandes sébacées.

Le marqueur biologique candidat selon l'invention et la description est préférablement un marqueur génique, un marqueur protéique, un marqueur lipidique ou un marqueur métabolique. Pour chacun de ces types de marqueurs, de nombreuses méthodes sont à la disposition de l'homme du métier pour mesurer l'expression dudit marqueur biologique et ainsi identifier une différence d'expression entre les cellules d'enfants de moins de 16 ans et les cellules d'adulte.

Dans un premier mode de réalisation, ledit marqueur est un marqueur génique ou un marqueur protéique.

Dans ce cas, la méthode de la description peut comprendre une ou plusieurs étapes intermédiaires entre le prélèvement de l'échantillon de cellules cutanées et la mesure de l'expression du marqueur biologique, lesdites étapes correspondant à l'extraction à partir dudit échantillon de cellules cutanées d'un échantillon d'ARNm (ou de l'ADNc correspondant) ou d'un échantillon de protéine. Celui-ci peut ensuite être directement utilisé pour mesurer l'expression du marqueur. La préparation ou l'extraction d'ARNm (ainsi que la rétrotranscription de celui-ci en ADNc) ou de protéines à partir d'un échantillon cellulaire ne sont que des procédures de routine bien connues de l'homme du métier.

Une fois qu'un échantillon d'ARNm (ou d'ADNc correspondant) ou de protéine est obtenu, l'expression du marqueur, au niveau soit des ARNm (c'est-à-dire dans l'ensemble des ARNm ou des ADNc présents dans l'échantillon), soit des protéines (c'est-à-dire dans l'ensemble des protéines présentes dans l'échantillon), peut être mesurée. La méthode utilisée pour ce faire dépend alors du type de transformation (ARNm, ADNc ou protéine) et du type d'échantillon disponible.

Quand l'expression du marqueur est mesurée au niveau de l'ARNm (ou d'ADNc correspondant), n'importe quelle technologie habituellement utilisée par l'homme du métier peut être mise en oeuvre. Ces technologies d'analyse du niveau d'expression des gènes, comme par exemple l'analyse du transcriptome, incluent des méthodes bien connues telles que la PCR (Polymerase Chain Reaction, si on part d'ADN), la RT-PCR (Reverse Transcription-PCR, si on part d'ARN) ou la RT-PCR quantitative ou encore les puces d'acides nucléiques (dont les puces à ADN et les puces à oligonucléotides) pour un plus haut débit.

Par « puces d'acides nucléiques », on entend ici plusieurs sondes d'acides nucléiques différentes qui sont attachées à un substrat, lequel peut être une micropuce, une lame de verre, ou une bille de la taille d'une microsphère. La micropuce peut être constituée de polymères, de plastiques, de résines, de polysaccharides, de silice ou d'un matériau à base de silice, de carbone, de métaux, de verre inorganique, ou de nitrocellulose. Les sondes peuvent être des acides nucléiques tels que les ADNc (« puce à ADNc »), les ARNm (« puce à ARNm ») ou des oligonucléotides (« puce à oligonucléotides »), lesdits oligonucléotides pouvant typiquement avoir une longueur comprise entre environ 25 et 60 nucléotides.

Pour déterminer le profil d'expression d'un gène particulier, un acide nucléique correspondant à tout ou partie dudit gène est marqué, puis mis en contact avec la puce dans des conditions d'hybridation, conduisant à la formation de complexes entre ledit acide nucléique cible marqué et les sondes attachées à la surface de la puce qui sont complémentaires de cet acide nucléique. La présence de complexes hybridés marqués est ensuite détectée.

Ces technologies permettent de suivre le niveau d'expression d'un gène en particulier ou de plusieurs gènes voire même de tous les gènes du génome (full genome ou full transcriptome) dans un échantillon biologique (cellules, tissus..). Ces technologies sont utilisées en routine par l'homme du métier et il n'est donc pas besoin de les détailler ici. Des exemples de mises en oeuvre du procédé basées sur l'analyse d'expression génique (puces à ADNc) et sur la PCR quantitative sont décrits dans la section expérimentale.

Alternativement, il est possible d'utiliser toute technologie actuelle ou future permettant de déterminer l'expression des gènes sur la base de la quantité d'ARNm dans l'échantillon. Par exemple, l'homme du métier peut mesurer l'expression d'un gène par hybridation avec une sonde d'acide nucléique marquée, comme par exemple par Northern blot (pour l'ARNm) ou par Southern blot (pour l'ADNc), mais aussi par des techniques telles que la méthode d'analyse sérielle de l'expression des gènes (SAGE) et ses dérivés, tels que LongSAGE, SuperSAGE, DeepSAGE, etc. Il est aussi possible d'utiliser des puces à tissu (aussi connues en tant que TMAs : « tissue microarrays »). Les tests habituellement employés avec les puces à tissu comprennent l'immunohistochimie et l'hybridation fluorescente in situ. Pour l'analyse au niveau de l'ARNm, les puces à tissu peuvent être couplées avec l'hybridation fluorescente in situ. Enfin, il est possible d'utiliser le séquençage massif en parallèle pour obtenir déterminer la quantité d'ARNm dans l'échantillon (RNA-Seq ou « Whole Transcriptome Shotgun Sequencing »). À cet effet, plusieurs méthodes de séquençage massif en parallèle sont disponibles. De telles méthodes sont décrites dans, par exemple, US 4,882,127, U.S. 4,849,077; U.S. 7,556,922; U.S. 6,723,513; WO 03/066896; WO 2007/111924; US 2008/0020392; WO 2006/084132; US 2009/0186349; US 2009/0181860; US 2009/0181385; US 2006/0275782; EP-B1-1141399; Shendure & Ji, Nat Biotechnol., 26(10): 1135-45. 2008; Pihlak et al., Nat Biotechnol., 26(6) : 676- 684, 2008 ; Fuller et al., Nature Biotechnol., 27(11): 1013-1023, 2009; Mardis, Genome Med., 1(4): 40, 2009; Metzker, Nature Rev. Genet., 11(1): 31-46, 2010.

Quand l'expression du marqueur est mesurée au niveau protéique, il est possible d'employer des anticorps spécifiques, en particulier dans des technologies bien connues telles que l'immunoprécipitation, l'immunohistologie, le western blot, le dot blot, l'ELISA ou l'ELISPOT, les puces à protéines, les puces à anticorps, ou les puces à tissu couplées à l'immunohistochimie. Parmi les autres techniques qui peuvent être utilisées sont comprises les techniques de FRET ou de BRET, les méthodes de microscopie ou d'histochimie, dont notamment les méthodes de microscopie confocale et de microscopie électronique, les méthodes basées sur l'utilisation d'une ou plusieurs longueurs d'onde d'excitation et d'une méthode optique adaptée, comme une méthode électrochimique (les techniques voltammétrie et d'ampérometrie), le microscope à force atomique, et les méthodes de radiofréquence, comme la spectroscopie résonance multipolaire, confocale et non-confocale, détection de fluorescence, luminescence, chemiluminescence, absorbance, réflectance, transmittance, and biréfringence ou index de réfraction (par exemple, par résonance des plasmons de surface, ou « surface plasmon resonance » en anglais, par ellipsometry, par méthode de miroir résonnant, tec.), cytométrie de flux, imagerie par résonance radioisotopique ou magnétique, analyse par électrophorèse en gel de polyacrylamide (SDS-PAGE); par spectrophotométrie HPLC-Mass, par chromatographie liquide/spectrophotométrie de masse/spectrométrie de masse (LC-MS/MS). Toutes ces techniques sont bien connues de l'homme du métier et il n'est pas nécessaire de les détailler ici.

Préférentiellement, le marqueur génique ou protéique candidat est sélectionné dans le groupe comprenant les marqueurs du métabolisme cellulaire, les marqueurs de la réponse au stress, les marqueurs de l'inflammation, les marqueurs de l'immunité, les marqueurs d'apoptose, les marqueurs de croissance/prolifération et du cycle cellulaire, les marqueurs de la signalisation cellulaire, les marqueurs de migration et de différenciation, les marqueurs de de la barrière épidermique, les marqueurs d'adhésion et les marqueurs des cellules souches pluripotentes de la peau.

L'homme du métier cherchant à déterminer à quelle classe appartient un marqueur génique ou protéique pourra facilement consulter la littérature scientifique pertinente ou se rapporter à des bases de données publiques telles que, par exemple, celles regroupées sur le site internet du National Center for Biotechnology Information (http://www.ncbi.nlm.nih.gov/guide/).

Plus préférentiellement, ledit marqueur du métabolisme énergétique cellulaire est IGFL3 (IGF-like family member 3), ledit marqueur de réponse au stress est GP3X (glutathione peroxidase 3), ledit marqueur de l'inflammation est sélectionné dans le groupe consistant en MGST1 (microsomal glutathione S-transferase 1), IL-1 (interleukine 1) et IL-8 (interleukine 8), ledit marqueur de l'immunité est sélectionné dans le groupe consistant en DEFB1 et DEFB4 (defensin beta1 et beta4), ledit marqueur du cycle cellulaire est CDKN1 (cyclin-dependent kinase inhibitor), ledit marqueur de différenciation est BARX2 (BARx Homeobox 2) ou SCEL (scielin), ledit marqueur de la barrière épidermique est sélectionné dans le groupe consistant en CLDN1 (claudin 1), IVL (involucrin), et KRT1 (keratin 1), ledit marqueur d'adhésion est CADM1 (cell adhésion molecule 1) et/ou ledit marqueur des cellules souches pluripotentes de la peau est sélectionné dans le groupe consistant FN1 (fibronectin 1), NID1 (nidogen 1), NOTCH1 (Notch homolog 1, translocation-associated), KRT19 (keratin 19), ITGB1BP1 (integrin beta1 binding protein), ITGA6 (integrin alpha6) et ITGB4 (integrin beta4).

Le gène CLDN 1 (NM_021101) code la protéine claudine 1 qui est un est un des composants les plus importants des jonctions serrées. L'involucrine, codée par le gène IVL (NM_005547) participe à la formation de l'enveloppe cornée des cornéocytes. Le gène KRT1 (NM_006121) code la kératine 1 constituant du réseau intracellulaire des kératinocytes. BARX2 (NM_003658) code un facteur transcription régulant l'adhésion pendant la formation de l'épiderme et la différenciation de celui-ci. Enfin, le produit du gène SCEL (NM_001160706 ; NM_003843 ; NM_144777), la scielline, participe à la régulation des protéines de l'enveloppe cornée.

GPX3 (NM_002084) code la glutathione peroxidase qui a un rôle de défense contre le stress oxydatif. DEFB1 (NM_005218) et DEFB4 (NM_004942) codant les beta défensines, sont plus particulièrement des gènes de l'immunité innée, dont les produits appartiennent à une même famille de peptides antimicrobiens.

Le gène MGST1 (NM_145792 ; NM_001267598 ; NM_145764 ; NM_001260511 ; NM_020300 ; NM_001260512 ; NM_145791) code une protéine de l'inflammation, la micorsomal glutathione transferase, laquelle est impliquée dans la production de médiateurs de l'inflammation.

Les gènes IL-1 (NM_000575; NM_000576 ; NM_001243211 ; NM_000577) et IL-8 (NM_000584) codent deux cytokines qui sont des médiateurs importants de l'inflammation.

FN1 (NM_212482 ; NM_002026 ; NM_212476 ; NM_212478 ; NM_212474 ; NM_054034) code la fibronectine 1, impliquée dans le processus d'adhésion des cellules souches. Le gène NOTCH1 (NM_017617) est impliqué dans le maintien des cellules souches. La protéine nidogène 1 ou entactine, codée par le gène NID1 (NM_002508), est un composant de la membrane basale impliqué chez les cellules souches, dans l'interaction avec la matrice. La protéine kératine 19, codée par le gène KRT19 (NM_002276) est un membre de la famille des kératines qui est spécifiquement exprimé dans les cellules souches. Enfin, ITGB1BP1 (NM_022334 ; NM_004763), ITGA6 (NM_001079818 ; NM_000210), ITGB4 (NM_000213 ; NM_001005731 ; NM_001005619) codent respectivement l'intégrine beta 1 binding protein, l'intégrine alpha 6 et l'intégrine beta 4, toutes essentielles pour l'adhésion des cellules souches à la matrice.

Des corps lamellaires situés dans la couche supérieure du *stratum granulosum* (couche granuleuse), les corps d'Odland, contiennent des stérols, des phospholipides et des glucosylcéramides, ainsi que des hydrolases spécifiques. Ces enzymes convertissent les phospholipides et les glucosylcéramides en acides gras libres et céramides qui forment avec le cholestérol et le cholestérolsulfate, une bicouche lamellaire intercellulaire. Celle-ci participe à la protection de la peau contre les agressions extérieures et au maintien d'un bon niveau d'eau intraépidermique (Jungerstend et al., Contact Dermitis, 58(5) : 255-262, 2008). Or, cette fonction de barrière de la peau évolue avec l'âge, en particulier entre la naissance et l'âge adulte (Fluhr et al., Exp Dermatol., 19(6) : 483-492, 2010). Outre les lipides de la bicouche lamellaire intercellulaire, la peau fabrique et secrète un mélange de corps gras appelé sébum comprenant des mono-, di-et triglycérides, des acides gras libres, des cires et cires estérifiées, des squalènes et des stérols. La sécrétion de sébum varie depuis la naissance jusqu'à l'âge adulte. Ainsi, cette sécrétion augmente durant la première semaine après la naissance, puis diminue avant d'augmenter de nouveau à la prépuberté (Fluhr et al., Exp Dermatol., 19(6) : 483-492, 2010).

Selon un autre mode de réalisation, le marqueur biologique selon la description est donc un marqueur lipidique.

En particulier, le marqueur lipidique selon la description peut être un marqueur lipidique de la couche cornée ou un marqueur lipidique du sébum.

Avantageusement, le marqueur lipidique selon la description est un marqueur lipidique de la couche cornée. De façon préférée, ledit lipide est choisi parmi les phospholipides, les céramides, les stérols et les acides gras libres.

Par « phospholipide », on entend ici tout lipide comprenant un groupement phosphate. Avantageusement, les phospholipides sont choisis dans le groupe constitué par les phosphoglycérides et les sphingomyélines. Un « phosphoglycéride » est ici un ester de glycérol où deux fonctions alcools sont estérifiées par des acides gras et la troisième par un acide phosphorique. Avantageusement, les acides gras sont saturés ou insaturés et contiennent de 14 à 20 atomes de carbone. De façon préférée, un phosphoglycéride est choisi dans le groupe constitué par la phosphatidyléthanolamine, la phosphatidylcholine, la phosphatidylsérine et le phosphatidylinositol. On entend par « sphingomyéline » une molécule constituée d'une base longue chaîne (sphingosine ou dihydrosphingosine), d'un acide gras amidifiant la fonction amine et d'un phosphate estérifiant la fonction alcool primaire et lui-même estérifié par un alcool aminé (choline).

Par « stérol », on entend ici un lipide appartenant à un sous-groupe de stéroïdes ayant un groupe hydroxyle libre, ainsi que tout dérivé dudit lipide. Les stérols peuvent être d'origine naturelle ou synthétique. Avantageusement, les stérols sont choisis parmi le cholestérol ou le cholestérolsulfate.

Un « céramide » est ici un lipide issu de la réaction d'amidification de la sphingosine et d'un acide gras. Un céramide est donc constitué d'une base sphingosine ou phytoshingosine liée par une liaison amide à des acides α-hydroxy, ω-hydroxy ou non hydroxy possédant des longueurs de chaîne hydrophobe variables. Dans le *stratum corneum* humain, 9 classes de céramides, dénommés CER 1 à 9, ont été identifiées (voir par exemple Dayan, Cosm & Toil, 121(1) : 37-44, 2006 ; Jungerstend et al., Contact Dermitis, 58(5) : 255-262, 2008 ; Farwick et al., Cosm & Toil, 124(2) : 63-72 ; Masukawa et al., J Lipid Res., 50(8): 1708-1719, 2009). Le céramide selon la description est choisi plus préférentiellement dans le groupe constitué par lesdits céramides CER1 à 9.

Le marqueur lipidique selon la description peut aussi être un marqueur lipidique du sébum. De façon préférée, un tel marqueur lipidique est choisi dans le groupe constitué par les mono-, di- et triglycérides, les acides gras libres, les cires et les cires estérifiées, les squalènes et les stérols.

Les « glycérides », ou acylglycérols ou glycérolipides, sont des esters d'acides gras et de glycérol. Selon qu'ils sont estérifiés par un, deux ou trois chaînes d'acides gras, ils sont appelés mono-, di- ou triglycérides.

On entend ici par « cires » des corps gras constitués de différents cérides, d'alcools et acides gras libres et souvent d'hydrocarbures saturés à longue chaîne. Par « céride », il est ici entendu des monoesters d'acides gras et d'alcools aliphatiques à longue chaîne ; les alcools des cérides sont en général des alcools primaires, à nombre pair de carbones, saturés et non ramifiés, tandis que la longueur des chaînes carbonées varie de 14 à 30 carbones pour l'acide gras et de 16 à 36 carbones pour l'alcool gras.

Un squalène est ici un triterpène, isoprénoïde à trente atomes de carbone et de cinquante atomes d'hydrogène, de formule : (E) 2,6,10,15,19,23-Hexaméthyl-2,6,10,14,18,22-tétracosahexène.

Enfin, la surface de la peau est recouverte d'un film protecteur qui permet à la peau de maintenir son hydratation et de se protéger contre les agressions extérieures. Ce film cutané de surface comprend, entre autres, un film hydrolipidique, lequel est constitué essentiellement de sueur, d'eau, de sébum et autres lipides. Ceux-ci comprennent des céramides, des triglycérides et des acides gras, en proportions à peu près égales. Les lipides du film hydrolipidique présentent une structure tridimensionnelle spécifique.

De nombreuses méthodes sont à la disposition de l'homme du métier pour mesurer la teneur en lipide dans un échantillon de cellules cutanées. Ces méthodes comprennent, entre autres, la chromatographie liquide en phase gazeuse (HPLC, voir par exemple Sullivan et al., Arch Ophthalmol., 120(12) : 1689-99, 2002), par exemple couplée à un détecteur évaporatif à diffraction de la lumière (HPLC-ESD, voir Nordbäck et al., J. High Resolut. Chromatogr., 22 : 483-486, 1999 ; Torres et al., J. Chromatogr. A., 1078 : 28-34, 2005) ; la chromatographie en couche mince (TLC, par exemple Downing et al., J Invest Dermatol., 77(4) : 358-360, 1981; Nordstrom et al., J Invest Dermatol., 87(2) : 260-263, 1986) ; la résonance magnétique nucléaire (NMR, voir par exemple Robosky et al., J Lipid Res., 49(3) : 686-692, 2008) ; la microspectroscopie confocale in vivo de Raman ; la spectrométrie de masse, la chromatographie en phase gazeuse couplée à la spectrométrie de masse (GC-MS, voir O'Neill et al., J Chromatogr Sci., 14(1) : 28-36, 1976) ; la chromatographie en phase liquide ultra-performante (UPLC, voir Rainville et al., J Proteome Res., 6(2):552-558, 2007 ; Castro-Perez et al., J Proteome Res., 10(9) : 4281-4290, 2011). On peut également analyser l'organisation de ces lipides dans la peau et plus particulièrement dans le stratum corneum (ou couche cornée), organisation lamellaire ou latérale, par des techniques par exemple de diffraction aux rayons X (Bouwstra et al., J Invest Dermatol., 97(6): 1005-1012, 1991 ; van Smeden et al., J Lipid Res., 52(6):1211-1221, 1991) ou par spectroscopie infra-rouge à transformée de Fourier (Gorcea et al., Int J Pharm. Nov 10, 2011.) ou par une analyse morphométrique en microscopie électronique (Daehnhardt-Pfeiffer et al., Skin Pharmacol Physiol., 25(3): 155-161, 2012) ou par une analyse en microscopie électronique de section vitreuse de peau combinée à une analyse moléculaire (Iwai et al., J Invest Dermatol., Apr 26, 2012).

Selon un autre mode de réalisation de la description, le marqueur biologique candidat est un marqueur métabolique.

Par « marqueur métabolique » on entend ici les marqueurs impliqués dans la régulation générale des procédés métaboliques de la peau, y compris le métabolisme énergétique. Le « métabolisme » correspond ici à l'ensemble des transformations moléculaires et énergétiques qui se déroulent de manière ininterrompue dans les cellules de la peau. Autrement dit, par « métabolisme », on entend désigner ici toutes les réactions par lesquelles, par exemple, les cellules de la peau produisent et utilisent l'énergie, maintiennent leur identité, se reproduisent, éliminent les composés toxiques, etc.

Les marqueurs métaboliques comprennent en particulier les métabolites de la peau qui sont présents de façon différentielle dans la peau des enfants de moins de 16 ans, et plus particulièrement des nouveau-nés, des nourrissons et/ou des enfants âgés de 2 à 16 ans, quand elle est comparée avec la peau d'adulte. Le terme « différentiel(le) » signifie ici une teneur différente dans l'échantillon mesuré que dans l'échantillon contrôle. Par exemple, un métabolite présent de façon différentielle dans la peau d'enfant (ou de nouveau-né, de nourrisson et/ou d'enfant âgé de 2 à 16 ans) peut être présent en quantités plus importantes dans ladite peau d'enfant que dans la peau d'adulte. Alternativement, ce métabolite peut être présent en quantités moins importantes dans la peau d'enfant (ou de nouveau-né, de nourrisson et/ou d'enfant âgé de 2 à 16 ans) que dans la peau d'adulte.

Les marqueurs métaboliques de la peau sont bien connus de l'homme du métier. Ils sont par exemple décrits dans Fluhr et al. (Exp Dermatol., 19(6) : 483-492, 2010) ou dans Jungerstend et al. (Contact Dermitis, 58(5) : 255-262, 2008). Les marqueurs biologiques selon la description comprennent les marqueurs de la construction de l'épiderme et de la fonction barrière de la peau, comme, par exemple l'éléidine ou les facteurs naturels d'hydratation, aussi appelés NMF (Natural Moisturizing Factors). Ceux-ci sont un mélange de substances hygroscopiques ayant la propriété de retenir l'eau (Fluhr et al., Exp Dermatol., 19(6) : 483-492, 2010). Certains de ces NMF sont issus de la protéolyse de la filaggrine selon une cascade de réactions impliquant des enzymes comme notamment la caspase 14 et la peptidylarginine deiminase (PAD1). D'autres molécules hygroscopiques épidermiques et dermiques comme l'acide hyaluronique contribuent à maintenir un bon niveau d'eau et à donner de la souplesse et du volume à la peau. Ce glycosaminoglycane est synthétisé par des hyaluronane synthases de type 1, 2 ou 3, alors qu'il est dégradé par des hyaluronidases. La fonction barrière de la peau est assurée essentiellement par la couche cornée et implique à la fois les lipides intercellulaires et les cornéocytes qui ont tous deux un rôle structurel et fonctionnel. Des enzymes accompagnent la synthèse et la maturation de ces lipides clés comme par exemple la betaglucocérébrosidase et la sphingomyelinase acide ; d'autres enzymes assurent aussi l'organisation des protéines constituant les cornéocytes comme notamment la tranglutaminase. Une fois que toutes les couches épidermiques sont constituées et que la différenciation est terminée, c'est le processus de desquamation qui permet l'élimination des cornéocytes impliquant des enzymes telles que les kallikréines 5 et 7. Sous l'épiderme, le derme peut subir un remaniement ou remodelage de la matrice extracellulaire mettant en jeu des métalloprotéinases et élastase.

Par ailleurs, il est connu que la concentration en hormones de la peau varie avec l'âge. Selon un autre aspect de l'invention et de la description, les marqueurs métaboliques de la peau sont donc des hormones, et en particulier les hormones sexuelles/stéroïdes comme les oestrogènes et les androgènes (par exemple œstradiol, progestérone et testostérone), les hormones de la glande surrénale qui sont aussi des stéroïdes (par exemple DHEA, cortisol), les hormones de la thyroïde (par exemple thyroxine et triiodothyrosine), les glucocorticoides (par exemple cortisol ou hydrocortisone), les neurohormones (par exemple pro-opiomelanocortine et adrenocorticotrophic hormone) etc.

Les marqueurs métaboliques la description peuvent aussi inclure des activités enzymatiques comme les enzymes antioxydantes, notamment la catalase, la glutathione peroxidase, superoxyde dismutase etc.

Les marqueurs métaboliques de la peau comprennent aussi les activités enzymatiques liées à l'élimination des composés toxiques exogènes (ou xénobiotiques) des cellules. Il est connu depuis longtemps que ce processus d'élimination, ou biotransformation, comporte généralement deux étapes : une étape de fonctionnalisation ou phase I, et une étape de conjugaison ou phase II. La biotransformation fait ainsi appel à deux types d'activités, les enzymes de phase I et les enzymes de phase II. Ces deux types d'activités enzymatiques sont présents dans les cellules de la peau et sont compris dans les marqueurs métaboliques de la peau (voir par exemple Ahmad et Mukhtar, J Invest Dermatol., 123 : 417-425, 2004 ; Cheung et al., J Dermatol Sci, 31 : 9-19, 2003 ; Gelardi et al., Toxicol in Vitro, 15 : 701-711, 2001 ; Hotchkiss, S.A.M., 1998. Dermal metabolism. In: Roberts, M.S., Walters, K.A. (Eds.) Dermal Absorption and Toxicity Assessment. Marcel Dekker Inc, New York, pp. 43-101; Janmohamed et al., Biochem Pharmacol, 62: 777-786,2001; Katiyar et al., J Invest Dermatol, 114 : 328-333,, 2000 ;. Mosset al., Food Chem Toxicol, 38 : 361-370, 2000 ; Oesch et al., Drug Metab Rev, 39 : 659-698, 2007 ; Oesch et al., Biochem Pharmacol, 27 : 17-20, 1978 ; Pillai et al., Pharm Res, 21 : 1146-1152, 2004 ; Raza et al., J Invest Dermatol, 96 : 463-467, 1991 ; Nordquist et Oreland, J Neural Transm, 114 : 713-716, 2007).

Avantageusement, le marqueur métabolique de la peau selon la description est une enzyme de phase I. Par « enzyme de phase I », on entend une enzyme permettant d'assurer une oxydation. Préférentiellement, l'enzyme de phase I est une enzyme de la famille des cytochromes P450, une flavine mono-oxygénase, une monoamine oxydase, une alcool déshydrogénase, une aldéhyde déshydrogénase, une estérase ou une époxyde hydrolase. Par « cytochrome P450 », on entend ici une enzyme constituée d'une partie protéique, l'apoprotéine, et d'un groupement prosthétique constitué d'une protoporphyrine liée à un atome de fer par quatre liaisons covalentes, ladite enzyme intervenant dans le métabolisme oxydatif de molécules très diverses. Une « flavine mono-oxygénase » est ici une enzyme qui fixe le flavine adénine dinucléotide (FAD) et catalyse avec l'aide du NADPH, l'oxygénation d'atomes nucléophiles comme le phosphore, l'azote ou le soufre qui sont présents dans de nombreux composés exogènes. On entend ici par « monoamine oxydase » une enzyme qui catalyse la désamination oxydative des amines primaires pourformer des aldéhydes. Les « alcools ou aldéhydes déshydrogénases » sont des enzymes qui catalysent l'oxydation des fonctions alcools en aldéhydes ou cétones, puis en acides carboxyliques. On entend ici par « estérase », des enzymes qui interviennent dans l'hydrolyse des fonctions esters et libèrent ainsi les fonctions alcool et acide carboxylique correspondantes. Une « époxyde hydrolase » est ici une enzyme réalisant la détoxification des époxydes par hydratation et formation de diols. Plus préférentiellement, l'enzyme de phase I est un cytochrome P450 choisi parmi CYP1A, CYP2C et CYP3A.

Alternativement, le marqueur métabolique de la peau selon la description est une enzyme de phase II. Par « enzyme de phase II », on entend une enzyme permettant d'assurer une conjugaison. Préférentiellement, une « enzyme de phase II » est une transférase, c'est-à-dire une enzyme catalysant la réaction entre un substrat nucléophile (alcool, acide carboxylique, amine ou thiol) et un substrat hydrosoluble électrophile. Plus préférentiellement, l'enzyme de phase II est une UDP-glucuronyltransférase, une sulfotransférase, une glutathion-S-transférase ou une N-acétyltransférase. On entend ici par « UDP-glucuronyltransférase » une enzyme qui permet la fixation de l'acide glucuronique sur un atome d'oxygène, d'azote ou de soufre d'une molécule pour former des composés glucuronides. Une « sulfotransférase » catalyse l'addition sur une molécule fonctionnalisée d'un groupement sulfate sous forme activée, ledit groupement sulfate étant donnée par le 3'-phosphoadénosine-5'-phosphosulfate (PAPS). Les « glutathion-S-transférases » (GSTs) sont une superfamille d'enzymes catalysant la conjugaison entre le glutathion réduit et des xénobiotiques électrophiles (composés aromatiques, époxydes, etc.). On entend ici, par « N-acétyltransférase » (NAT) une enzyme responsable de la biotransformation par acétylation de divers composés (aminés aromatiques, sulfonamides, hydrazines, etc.).

Les marqueurs métaboliques de la description comprennent également les marqueurs liés à la pigmentation de la peau.

La pigmentation de la peau résulte de la présence de grains de mélanine dans les kératinocytes. Selon un premier aspect, le marqueur lié à la pigmentation de la peau selon l'invention et la description est donc une mélanine. La mélanine est le pigment le plus important de la peau : le taux de mélanine dans la peau détermine sa couleur (claire, mate...) et assure une plus ou moins grande protection vis à vis des rayons ultraviolets. Par « mélanine », on entend ici aussi bien la phéomélanine que l'eumélanine. La « phéomélanine » ou « phémélanine » est un pigment jaune ou rouge-brun, principalement composé de dérivés benzothiazine riches en soufre. Par « eumélanine », on entend ici un copolymère fortement hétérogène consistant d'unités de DHI (5,6-dihydroxyindole) et de DHICA (acide 5,6-dihydroxyindole-2-carboxylique) et formant un pigment brun-noir, pauvre en soufre. L'eumélanine et la phéomélanine sont bien connues de l'homme du métier (voir par exemple Bertolo et al., m/s, 17(2) : 177-185, 2001 ; Ito et Wakamatsu, Pigment Cell Res, 16(5) : 523-531, 2003 ; Ebanks et al., Int J Mol Sci, 10 : 4066-4087, 2009).

Selon un second aspect, le marqueur lié à la pigmentation de la peau selon la description est une activité enzymatique régulant la synthèse d'une mélanine. Avantageusement, ledit marqueur est une activité de type « tyrosinase ». Une activité tyrosinase est ici une activité enzymatique qui est capable de catalyser l'hydroxylation de la tyrosine en dihydroxyphénylalanine (DOPA) et de la DOPA en dopaquinone. Un exemple de tyrosinase correspond par exemple au produit du locus *albino* de la souris, ainsi qu'à ses homologues dans les autres espèces de mammifères. La réaction catalysée par la tyrosinase est commune à la biosynthèse des eumélanines et à celle de la phéomélanine (Bertolo et al., m/s, 17(2) : 177-185, 2001 ; Ebanks et al., Int J Mol Sci, 10 : 4066-4087, 2009).

Alternativement, le marqueur lié à la pigmentation de la peau est une activité enzymatique spécifique de la synthèse de la phéomélanine ou de l'eumélanine. Préférentiellement, ladite activité enzymatique est spécifique de la synthèse de l'eumélanine. Plus préférentiellement, ladite activité enzymatique est une activité de type DHICAoxydase (ou TRP-1 ou TYRP1 ou gp75) ou de type DOPAchrome tautomérase (ou TRP-2 ou TYRP2). On entend ici par DHICAoxydase une activité enzymatique capable de catalyser l'oxydation de l'acide 5,6-dihydroxyindole-2-carboxylique (DHICA) en acide indole 5,6-quinone-2-carboxylique. Une telle activité est, par exemple, portée par une protéine homologue du produit du locus murin *brown.* La DOPAchrome tautomérase est une activité enzymatique capable de catalyser l'oxydation du dopachrome en DHICA qui est, par exemple, codée par un gène homologue du gène murin *slaty.*

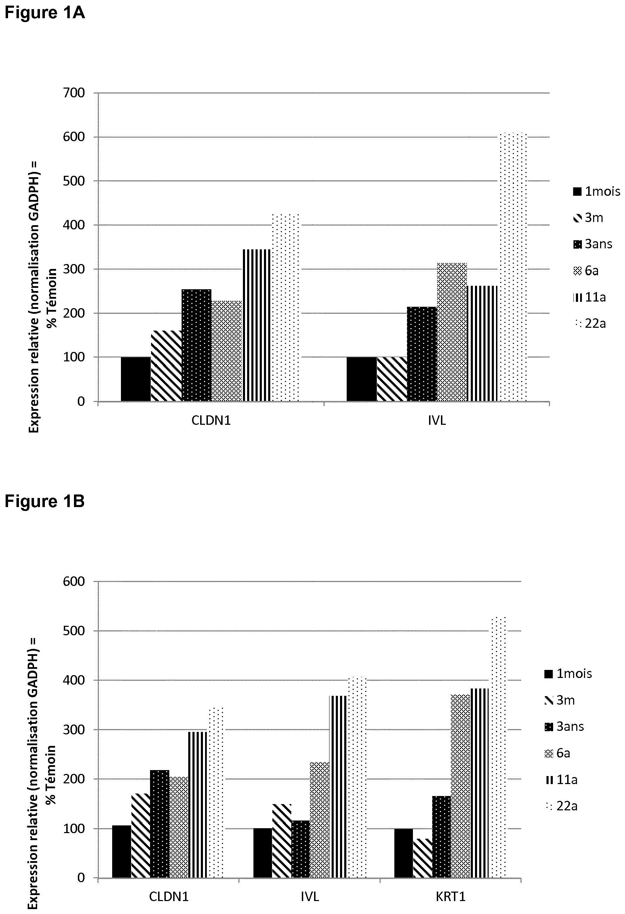

Il est entendu ici que les produits des réactions catalysés par les différentes activités enzymatiques entrent aussi dans la définition des marqueurs de la pigmentation de la peau. Ainsi, la DOPA, la dopaquinone, la leucodopachrome, la dopachrome, la DHI, l'indole-5,6-quinone, la DHICA, l'acide indole 5,6-quinone-2-carboxylique sont-ils tous des marqueurs biologiques de la peau, tout comme la cystéinyl-dopa et la 1,4-benzothiazinylalanine (voir par exemple Figure 1 de Bertolo et al., m/s, 17(2) : 177-185, 2001).

L'hypoderme comprend des cellules spécialisées dans la synthèse et le stockage des triglycérides, les adipocytes. L'hypoderme peut ainsi comprendre des adipocytes blancs, constituant la graisse blanche ou le tissu adipeux blanc, et des adipocytes bruns, formant la graisse brune ou tissu adipeux brun. Cette dernière est surtout présente au début de la vie.

Sont donc aussi compris dans les marqueurs biologiques selon la description les marqueurs liés aux adipocytes. En particulier, les marqueurs selon la description comprennent le rapport entre le nombre d'adipocytes blancs et le nombre d'adipocytes bruns. Les adipocytes de la graisse blanche sont des cellules sphériques, d'un diamètre d'environ une centaine de micromètres, dont le cytoplasme renferme une volumineuse vacuole lipidique unique (triglycérides), entourée par une mince couronne cytoplasmique contenant un appareil de Golgi, du réticulum endoplasmique granulaire, du réticulum endoplasmique lisse et des mitochondries. Contrairement aux adipocytes blancs, les adipocytes bruns ont un noyau central et un cytoplasme rempli de nombreuses petites vacuoles lipidiques (la cellule est dite multiloculaire) et de mitochondries.

Les adipocytes blancs sécrètent de nombreux peptides, appelés collectivement les adipocytokines (Avram et al., J Am Acad Dermatol, 53(4) : 671-683, 2005) lesquels comprennent, entre autres, la leptine, l'adipsine, l'adiponectine, et la protéine ASP (acylation stimulating protein). Dans un mode de réalisation préféré, les marqueurs liés aux adipocytes de l'invention et de la description sont des adipocytokines ; plus préférentiellement, lesdits marqueurs sont choisis parmi la leptine, l'adipsine, l'adiponectine, la résistine,et la protéine ASP. Par « leptine », on entend ici une protéine de 16 kDa, codée par le gène Ob, et qui régule les réserves de graisses dans l'organisme et l'appétit en contrôlant la sensation de satiété. Avantageusement, la leptine a la séquence peptidique représentée par NP_000221.1. On entend par « adipsine » ou « Factor D » ou « complement factor D(CFD) » une protéine de 253 résidus ayant une activité sérine protéase et codée par le gène *CFD.* Avantageusement, l'adipsine a la séquence protéique représentée par NP_001919. On entend par « adiponectine » ou « AdipoQ », « Acr30 » (adipocyte complement-related protein of 30 kD), « APM1 » (adipose most abundant gene transcript 1), ou « GBP28 » (gelatin-binding protein of 28 kD), une protéine de 244 acides aminés impliquée, entre autres, dans la régulation du métabolisme des lipides et du glucose. Avantageusement, cette protéine possède la séquence d'acides aminés représentée par NP_001171271.1. La « résisitine » ou « ADSF » (adipose tissue-specific secretory factor) ou « XCP1 » (C/EBP-epsilon-regulated myeloid-specific secreted cysteine-rich protein) est une protéine de 108 acides aminés codée par le gène *RSTN.* Avantageusement la protéine résistine possède la séquence protéique NP_001180303.1. La protéine « ASP », telle qu'on l'entend ici, est un produit de clivage du facteur de complément C3 (NP_000055.2) qui est spécifique des adipocytes et qui régule l'entrée dans ces cellules du glucose et le stockage des acides gras non estérifiés.

Les adipocytes blancs peuvent libérer de l'énergie sous forme d'acides gras libres à partir des triglycérides stockés : c'est la lipolyse. Selon un autre mode de réalisation préféré, les marqueurs liés aux adipocytes de la description comprennent les activités enzymatiques liées à la lipolyse, c'est-à-dire à l'hydrolyse des triglycérides. Préférentiellement, ladite activité enzymatique est une activité de type lipase. Une « lipase » est une enzyme hydrosoluble capable d'effectuer l'hydrolyse de fonctions esters et spécialisée dans la transformation de triglycéride en glycérol et en acides gras. La lipase est plus préférentiellement la lipase hormono-sensible, la monoglycéride lipase et l'adipose triglycéride lipase (Lafontan et Langin, Prog Lipid Res, 48(5) : 275-297, 2009). La « lipase hormono-sensible » telle qu'on l'entend ici est une enzyme capable d'hydrolyser les triglycérides en diglycérides et les diglycérides en monoglycérides et dont l'activité est régulée par le taux intracellulaire d'AMPc à travers une phosphorylation par la protéine kinase A (PKA). Un exemple de lipase hormono-sensible correspond à la protéine HSL humaine, dont la séquence est donnée par NP_005348.2. On entend ici par « monoglycéride lipase » une enzyme associée à la membrane capable d'hydrolyser les monoglycérides en glycérol et acide gras. La monoglycéride lipase humaine correspond à la protéine MAGL, MGL ou MGLL (NP_001243514). Une «adipose triglycéride lipase » est ici une enzyme capable d'hydrolyser les triglycérides, mais uniquement les triglycérides (et donc pas les diglycérides et/ou les monoglycérides), et n'est pas phosphorylée par la PKA. On pourra, par exemple, se rapporter à la protéine ATGL humaine, qui a pour séquence celle correspondant au numéro d'identification NP_065109.1.

Comme indiqué plus haut, les marqueurs biologiques sont bien connus de l'homme du métier. Les méthodes pour doser lesdits marqueurs biologiques ont été décrites et utilisées en routine en laboratoire depuis de nombreuses années et il n'est donc pas besoin de les détailler plus en détail ici.

Avantageusement, l'expression du marqueur candidat est normalisée par rapport à l'expression d'un marqueur témoin. Un « marqueur témoin » est ici un marqueur dont l'expression est identique quels que soient le type cellulaire considéré et l'âge du donneur. Autrement dit, le marqueur témoin est exprimé au même niveau chez l'enfant, et en particulier chez le nouveau-né, le nourrisson et/ou l'enfant entre 2 et 16 ans, et l'adulte.

En particulier, quand le marqueur candidat est un marqueur génique ou un marqueur protéique, le marqueur témoin est un gène qui est exprimé dans tous les types cellulaires, indépendamment de l'âge du sujet, ou son produit protéique. En particulier, ledit marqueur témoin est un gène de ménage ou le produit protéique dudit gène de ménage. Un gène de ménage est un gène qui est exprimé dans tous les types cellulaires et qui fournit une fonction de base qui est nécessaire pour la survie de la cellule. Une liste de gènes de ménage humain peut par exemple être trouvée dans Eisenberg et al. (Trends in Genetics 19: 362-365, 2003). Un gène de ménage préféré est un gène sélectionné dans le groupe constitué de B2M, TFRC, YWHAZ, RPLO, 18S, GUSB, UBC, TBP, GAPDH, PPIA, POLR2A, ACTB, PGK1, HPRT1, IPO8 et HMBS.

Il est clair pour l'homme du métier que le procédé de la description présente l'avantage de permettre l'isolement et la caractérisation aisés d'agents actifs, de matières premières cosmétiques et/ou de formulations cosmétiques. En particulier, le procédé de la description permet de vérifier facilement la tolérance, la dermopénétration et l'efficacité d'un agent actif, d'une composition dermo-cosmétique ou d'une formulation cosmétique. Par exemple, on peut désirer, dans certains cas, de vérifier que ces agents, ces compositions ou ces formulations sont bien tolérés et n'induisent pas l'expression de marqueurs indiquant un stress, comme par exemple les marqueurs liés à l'inflammation.

Il est donc aussi ici décrit un procédé d'évaluation de la tolérance d'un agent actif, d'une composition dermo-cosmétique ou d'une formulation cosmétique, comprenant les étapes suivantes :
a) obtenir au moins un échantillon (A) de cellules cutanées d'enfant ;
b) mettre en contact un agent actif, d'une composition dermo-cosmétique ou d'une formulation cosmétique, avec l'échantillon (A) ;
c) mesurer le niveau d'expression d'au moins un marqueur biologique caractérisant une peau d'enfant identifié selon le procédé de l'invention et de la description ;
d) mesurer le niveau d'expression dudit marqueur biologique dans un échantillon témoin ;
e) calculer le rapport entre le niveau d'expression de l'étape c) et le niveau d'expression d'étape d), et
f) déterminer si ledit agent actif, la composition dermo-cosmétique ou la formulation cosmétique est bien tolérée par la peau d'enfant.

L'invention concerne en particulier un procédé tel que revendiqué dans la revendication 11. Dans l'invention, le donneur de l'échantillon (A) est plus particulièrement un donneur ayant un âge compris entre 0 et 1 mois, entre 1 mois et 2 ans ou entre 2 ans et 16 ans. Autrement dit, le donneur de l'échantillon (A) est choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans.

L'échantillon témoin est par exemple un échantillon qui n'a pas été en contact avec l'agent actif, la composition dermo-cosmétique ou la formulation cosmétique, ce qui permet d'effectuer une comparaison significative entre le niveau d'expression de l'étape c) et celui de l'étape d). Par exemple, l'échantillon (A) qui n'a pas été traité par l'actif, la composition ou la formulation peut être utilisé comme témoin. Dans ce cas, le niveau d'expression du marqueur biologique est mesuré dans l'échantillon (A) avant et après avoir été mis en contact avec l'agent actif, la composition dermo-cosmétique ou la formulation cosmétique.

L'agent actif est un agent actif qui est bien toléré par la peau d'enfant si ledit agent actif ne module pas l'expression du marqueur biologique. De la même façon, la composition dermo-cosmétique ou la formulation cosmétique sont bien tolérées si l'expression du marqueur biologique n'est pas modulée par leur addition sur l'échantillon (A). Ladite modulation peut correspondre, selon les cas, et en particulier selon la nature du marqueur biologique, à une augmentation ou à une diminution de l'expression dudit marqueur. En particulier, l'expression des marqueurs de l'inflammation est connue pour être augmentée quand la peau d'enfant est agressée. En revanche, l'expression de ces marqueurs de l'inflammation n'est pas affectée par des actifs, des compositions ou des formulations bien tolérés, comme le montrent les résultats expérimentaux. De préférence, le marqueur biologique de l'étape c) est un marqueur de l'inflammation. Plus préférablement, le marqueur biologique de l'étape c) est IL1 ou IL8. Dans l'invention, le marqueur biologique est un marqueur de l'inflammation choisi parmi IL1 ou IL8.

Le procédé peut en outre comporter une comparaison de la viabilité cellulaire dans l'échantillon traité avec l'actif, la composition ou la formulation et dans l'échantillon témoin. Dans ce cas, l'actif, la composition dermo-cosmétique ou la formulation cosmétique est bien toléré par la peau d'enfants si la viabilité cellulaire de l'échantillon n'est pas affectée par la présence de l'agent actif, la composition dermo-cosmétique ou la formulation cosmétique.

Selon un autre mode de réalisation préféré, le procédé de la description comprend donc une étape supplémentaire de détermination de la viabilité cellulaire dans l'échantillon (A) traité avec l'agent actif, la composition dermo-cosmétique ou la formulation cosmétique, de détermination de la viabilité cellulaire dans l'échantillon témoin et de comparaison entre les deux.

De nombreux tests pour déterminer la viabilité cellulaire sont disponibles pour l'homme du métier et sont couramment utilisés en cosmétologie. On citera en particulier le test MTT, décrit par exemple dans Mosman et al. (J Immunol Methods, 65(1-2) : 55-63, 1983).

Il peut aussi être intéressant de rechercher des agents qui entrainent une expression accrue de marqueurs caractéristiques d'une tranche d'âge différente, comme, par exemple, des gènes et/ou des protéines de la barrière.

Dans un autre aspect, le procédé permet donc d'isoler des agents actifs ayant un effet sur la peau d'enfant et, plus particulièrement, sur la peau de nouveau-né, de nourrisson et/ou d'enfant âgé de 2 à 16 ans. L'identification de marqueurs biologiques permet d'identifier les agents actifs modulant ou non l'expression de ces marqueurs.

Il est donc ici décrit un procédé d'identification d'un agent actif pour la préparation d'une composition dermo-cosmétique pour enfant, ledit procédé comprenant les étapes suivantes :
a) obtenir au moins un échantillon (A) de cellules cutanées d'enfant ;
b) mettre en contact un agent actif candidat avec l'échantillon (A) ;
c) mesurer le niveau d'expression d'au moins un marqueur biologique caractérisant une peau d'enfant identifié selon le procédé de l'invention et de la description ;
d) mesurer le niveau d'expression dudit marqueur biologique dans un échantillon témoin ;
e) calculer le rapport entre le niveau d'expression de l'étape c) et le niveau d'expression de l'étape d), et
f) déterminer si ledit agent actif candidat est un agent actif pour la préparation d'une composition dermo-cosmétique de la peau d'enfant.

De préférence, le donneur de l'échantillon (A) est plus particulièrement un donneur ayant un âge compris entre 0 et 1 mois, entre 1 mois et 2 ans ou entre 2 ans et 16 ans. Autrement dit, dans ce cas, le donneur de l'échantillon (A) est choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans.

Il est donc également ici décrit un procédé d'identification d'un agent actif pour la préparation d'une composition dermo-cosmétique pour enfant, ledit procédé comprenant les étapes suivantes :
a) obtenir au moins un échantillon (A) de cellules cutanées, ledit échantillon provenant d'un donneur, ledit donneur étant choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans ;
b) mettre en contact un agent actif candidat avec l'échantillon (A) ;
c) mesurer le niveau d'expression d'au moins un marqueur biologique caractérisant une peau d'enfant identifié selon le procédé de l'invention et de la description ;
d) mesurer le niveau d'expression dudit marqueur biologique dans un échantillon témoin ;
e) calculer le rapport entre le niveau d'expression de l'étape c) et le niveau d'expression de l'étape d), et
f) déterminer si ledit agent actif candidat est un agent actif pour la préparation d'une composition dermo-cosmétique de la peau d'enfant.

L'échantillon témoin est un échantillon qui n'a pas été en contact avec l'agent actif candidat, ce qui permet d'effectuer une comparaison significative entre le niveau d'expression de l'étape c) et celui de l'étape d). Par exemple, l'échantillon (A) qui n'a pas été traité par l'agent actif candidat peut être utilisé comme témoin. Dans ce cas, le niveau d'expression du marqueur biologique est mesuré dans l'échantillon (A) avant et après avoir été mis en contact avec l'agent actif candidat.

L'agent actif candidat est un agent actif pour la préparation d'une composition dermo-cosmétique de peau d'enfant si ledit agent actif permet de moduler l'expression du marqueur biologique. Cette modulation peut correspondre, selon les cas, et en particulier selon la nature du marqueur biologique, à une augmentation ou à une diminution de l'expression dudit marqueur. En particulier, il peut être intéressant d'isoler des agents actifs stimulant l'expression des marqueurs de la barrière cutanée ou limitant les marqueurs de l'inflammation.

Dans un autre aspect, le procédé permet l'isolement de matière première pouvant être utilisées dans la mise au point de formulations pour la peau de l'enfant et, plus particulièrement, pour la peau de nouveau-né, de nourrisson et/ou d'enfant âgé de 2 à 16 ans.

Une formulation est ici une préparation résultant d'un mélange de différentes matières premières, afin de répondre à une demande exprimée, en général, en termes de propriétés. Les formulations peuvent être utilisées dans le domaine cosmétique, pharmaceutique, alimentaire et/ou nutraceutique. Elles peuvent être utilisées chez l'homme ou l'animal, par application topique ou orale.

Le procédé permet ainsi d'identifier des matières premières améliorant la tolérance et la dermo-pénétration. L'identification de marqueurs biologiques permet d'identifier les matières premières modulant ou non l'expression de ces marqueurs. Il est donc également ici décrit un procédé d'identification d'une matière première pouvant être utilisée pour la préparation d'une formulation cosmétique, pharmaceutique, alimentaire et/ou nutraceutique pour enfant, ledit procédé comprenant les étapes suivantes :
a) obtenir au moins un échantillon (A) de cellules cutanées, ledit échantillon provenant d'un donneur ayant un âge inférieur à 16 ans ;
b) mettre en contact une matière première candidate avec l'échantillon (A) ;
c) mesurer le niveau d'expression d'au moins un marqueur biologique caractérisant une peau d'enfant identifié selon le procédé de l'invention et de la description ;
d) mesurer le niveau d'expression dudit marqueur biologique dans un échantillon témoin ;
e) calculer le rapport entre le niveau d'expression de l'étape c) et le niveau d'expression de l'étape d), et
f) déterminer si ladite matière première candidate est une matière première pour la préparation d'une formulation cosmétique, pharmaceutique, alimentaire et/ou nutraceutique pour enfant.

De préférence, le donneur de l'échantillon (A) est plus particulièrement un donneur ayant un âge compris entre 0 et 1 mois, entre 1 mois et 2 ans ou entre 2 ans et 16 ans. Autrement dit, dans ce cas, le donneur de l'échantillon (A) est choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans.

Il est donc également ici décrit un procédé d'identification d'une matière première pouvant être utilisée pour la préparation d'une formulation cosmétique, pharmaceutique, alimentaire et/ou nutraceutique pour enfant, ledit procédé comprenant les étapes suivantes :
a) obtenir au moins un échantillon (A) de cellules cutanées, ledit échantillon provenant d'un donneur, ledit donneur étant choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans ;
b) mettre en contact une matière première candidate avec l'échantillon (A) ;
c) mesurer le niveau d'expression d'au moins un marqueur biologique caractérisant une peau d'enfant identifié selon le procédé de l'invention et de la description ;
d) mesurer le niveau d'expression dudit marqueur biologique dans un échantillon témoin ;
e) calculer le rapport entre le niveau d'expression de l'étape c) et le niveau d'expression de l'étape d), et
f) déterminer si ladite matière première candidate est une matière première pour la préparation d'une formulation cosmétique, pharmaceutique, alimentaire et/ou nutraceutique pour enfant.

L'échantillon témoin est un échantillon qui n'a pas été en contact avec la matière première candidate, ce qui permet d'effectuer une comparaison significative entre le niveau d'expression de l'étape c) et celui de l'étape d). Par exemple, l'échantillon (A) qui n'a pas été traité par la matière première candidate peut être utilisé comme témoin. Dans ce cas, le niveau d'expression du marqueur biologique est mesuré dans l'échantillon (A) avant et après avoir été mis en contact avec la matière première candidate.

Il est clair que le procédé permet ainsi, non seulement d'isoler et de caractériser des matières premières pouvant être utilisées dans des formulations, mais en outre de tester des formulations déjà constituées et d'identifier celles qui présentent des qualités de tolérance, d'efficacité, de toxicologie et de dermo-pénétration optimales vis-à-vis de la peau d'enfant. L'homme du métier comprendra en effet aisément qu'il ne suffit que de mesurer l'expression d'un ou plusieurs marqueurs biologiques pour déterminer si une formulation peut être utilisée sur la peau d'enfant.

Il est donc également ici décrit un procédé d'identification d'une formulation cosmétique, pharmaceutique, alimentaire et/ou nutraceutique pour enfant, ledit procédé comprenant les étapes suivantes :
a) obtenir au moins un échantillon (A) de cellules cutanées, ledit échantillon provenant d'un donneur ayant un âge inférieur à 16 ans ;
b) mettre en contact une formulation candidate avec l'échantillon (A) ;
c) mesurer le niveau d'expression d'au moins un marqueur biologique caractérisant une peau d'enfant identifié selon le procédé de l'invention et de la description ;
d) mesurer le niveau d'expression dudit marqueur biologique dans un échantillon témoin ;
e) calculer le rapport entre le niveau d'expression de l'étape c) et le niveau d'expression de l'étape d), et
f) déterminer si ladite formulation cosmétique candidate est une formulation cosmétique, pharmaceutique, alimentaire et/ou nutraceutique pour enfant.

De préférence, le donneur de l'échantillon (A) est plus particulièrement un donneur ayant un âge compris entre 0 et 1 mois, entre 1 mois et 2 ans ou entre 2 ans et 16 ans. Autrement dit, dans ce cas, le donneur de l'échantillon (A) est choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans.

Il est donc également ici décrit un procédé d'identification d'une formulation cosmétique, pharmaceutique, alimentaire et/ou nutraceutique pour enfant, ledit procédé comprenant les étapes suivantes :
a) obtenir au moins un échantillon (A) de cellules cutanées, ledit échantillon provenant d'un donneur, ledit donneur étant choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans ;
b) mettre en contact une formulation candidate avec l'échantillon (A) ;
c) mesurer le niveau d'expression d'au moins un marqueur biologique caractérisant une peau d'enfant identifié selon le procédé de l'invention et de la description ;
d) mesurer le niveau d'expression dudit marqueur biologique dans un échantillon témoin ;
e) calculer le rapport entre le niveau d'expression de l'étape c) et le niveau d'expression de l'étape d), et
f) déterminer si ladite formulation cosmétique candidate est une formulation cosmétique, pharmaceutique, alimentaire et/ou nutraceutique pour enfant.

L'échantillon témoin est un échantillon qui n'a pas été en contact avec la formulation candidate, ce qui permet d'effectuer une comparaison significative entre le niveau d'expression de l'étape c) et celui de l'étape d). Par exemple, l'échantillon (A) qui n'a pas été traité par la formulation cosmétique candidate peut être utilisé comme témoin. Dans ce cas, le niveau d'expression du marqueur biologique est mesuré dans l'échantillon (A) avant et après avoir été mis en contact avec la formulation candidate.

Selon un autre aspect, les marqueurs biologiques caractérisant une peau d'enfant permettent de caractériser les troubles cutanés affectant les enfants. Plus particulièrement, il est possible de caractériser, à l'aide desdits marqueurs biologiques, les maladies de peau affectant les nouveau-nés, les nourrissons et/ou les enfants dont l'âge est compris entre 2 et 16 ans.

Par « troubles cutanés », il est ici entendu toutes les réactions anormales qui peuvent subvenir sur la peau d'un individu. Ces conditions touchent aussi bien la peau en elle-même (c'est-à-dire l'épiderme, le derme et/ou l'hypoderme), que les pores de la peau, les glandes sudoripares et sébacées qui y sont annexées, les cheveux ou les ongles.

Les troubles cutanés se traduisent ici par des lésions, ce qui correspond à une peau endommagée ou en mauvais état. La peau endommagée comprend par exemple la peau sensible réactive, la peau sèche, la peau endommagée par le soleil, par les radiations, par le froid, par le stress ou par la pollution, par une allergie, par un urticaire, par un eczéma et d'autres formes de dermatites comme la dermatite atopique, l'impétigo, la dermatite irritative, en particulier la dermite irritative du siège ou érythème fessier, la dermatite de contact, la dermite séborrhéique de la peau et du cuir chevelu (croûte de lait), le psoriasis, la maladie de Lainer-Moussous, ou encore par des plaies ou des brûlures. Par trouble cutané, on entend donc des troubles aussi divers que les dartres, les angiomes (y compris tubéreux, sous-cutanés ou plans), les hémangiomes, l'acné du nourrisson, l'acné de l'adolescent, les ichtyoses (par exemple vulgaris, congénitale, arlequin...) etc. Un trouble cutané peut aussi être causé ou exacerbé par une infection externe par exemple d'origine parasitaire, virale, bactérienne ou fongique. Sont ainsi incluses en particulier dans les troubles cutanés les verrues, le prurigo strophulus, la gale, la pédiculose du cuir chevelu, ou encore les mycoses. Ces dernières sont des parasitoses causées par la prolifération de champignons microscopiques parasites de l'organisme. Parmi les mycoses les plus fréquentes, on peut citer les candidoses et les pityrosporoses, qui sont provoquées par la prolifération de levures de la peau.

En particulier, il est ici décrit un procédé d'identification d'au moins un marqueur biologique d'un trouble cutané affectant les enfants, ledit procédé comprenant les étapes suivantes :
a) obtenir au moins un échantillon (A') de cellules cutanées d'enfant, lesdites cellules provenant d'un sujet affecté par ledit trouble cutané;
b) obtenir au moins un échantillon témoin (B) de cellules cutanées d'enfant, lesdites cellules provenant d'un sujet sain ;
c) mesurer le niveau d'expression d'un marqueur biologique candidat dans l'échantillon de l'étape a), ledit marqueur biologique candidat étant un marqueur biologique caractérisant une peau d'enfant tel qu'identifié selon le procédé de l'invention et de la description décrit plus haut ;
d) mesurer le niveau d'expression dudit marqueur biologique candidat dans l'échantillon de l'étape b),
e) calculer le rapport entre le niveau d'expression de l'étape a) et le niveau d'expression de l'étape b), et
f) déterminer si le marqueur biologique candidat est un marqueur biologique d'un trouble cutané affectant les enfants.

De préférence, l'échantillon (A') provient d'un donneur qui est plus particulièrement un donneur ayant un âge compris entre 0 et 1 mois, entre 1 mois et 2 ans ou entre 2 ans et 16 ans. Autrement dit, dans ce cas, le donneur de l'échantillon (A') est choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans.

Préférentiellement, ledit marqueur biologique candidat est un marqueur biologique d'un trouble cutané si son niveau d'expression est différent dans une peau saine et dans une peau présentant les caractères cliniques dudit trouble cutané. De façon plus préférée, ledit marqueur caractérise un trouble cutané si ledit marqueur est exprimé de façon différentielle dans le sujet affecté par le trouble cutané et le sujet sain. Cela se traduit par un rapport à l'étape e) qui est différent de 1.

Comme la personne du métier s'en rendra compte sans peine, l'identification de marqueurs spécifiques de maladies de peau de l'enfant (et en particulier du nouveau-né, du nourrisson et/ou de l'enfant âgé de 2 à 16 ans) présente l'avantage immédiat de permettre l'isolement d'agents actifs pour traiter lesdites maladies de peau.

Il est donc aussi ici décrit un procédé d'identification d'un agent actif pour traiter les troubles cutanés des enfants, ledit procédé comprenant les étapes suivantes :
a) obtenir au moins un échantillon (A') de cellules cutanées d'enfant, lesdites cellules provenant d'un sujet affecté par ledit trouble cutané;
b) mettre en contact un agent actif candidat avec la surface dudit échantillon (A') ;
c) mesurer dans l'échantillon (A') de l'étape b) le niveau d'expression d'au moins un marqueur biologique dudit trouble cutané affectant les enfants identifié selon le procédé décrit plus haut ;
d) mesurer le niveau d'expression dudit marqueur biologique dans au moins un échantillon témoin ;
e) calculer le rapport entre le niveau d'expression de l'étape c) et le niveau d'expression de l'étape d), et
f) déterminer si ledit agent actif candidat est un agent actif pour traiter les troubles cutanés d'enfant.

De préférence, l'échantillon (A') provient d'un donneur qui est plus particulièrement un donneur ayant un âge compris entre 0 et 1 mois, entre 1 mois et 2 ans ou entre 2 ans et 16 ans. Autrement dit, dans ce cas, le donneur de l'échantillon (A') est choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans.

De façon avantageuse, un agent actif pour traiter ledit trouble cutané est capable de moduler l'expression de dudit marqueur biologique dudit trouble cutané. Un candidat sera donc un agent actif s'il conduit à une expression dudit marqueur biologique différente dans les cellules (A') non traitées avec ledit candidat et dans les cellules traitées. Dans ce cas, le témoin de l'étape d) sera avantageusement un échantillon (A') non traité. De façon encore plus avantageuse, ledit agent actif permet de moduler le niveau d'expression dudit marqueur biologique dans l'échantillon traité de façon à ce qu'il soit proche de celui observé dans un échantillon sain. Dans ce cas, l'étape d) comprendra la mesure du niveau d'expression dudit marqueur biologique dans au moins deux échantillons témoins, l'un correspondant à un échantillon (A') non traité et l'autre à un échantillon (B) sain. Si le marqueur candidat est un agent actif pour traiter les troubles cutanés, on devrait alors observer que le rapport entre les niveaux d'expression de (A') traité et (A') non traité est différent de 1, tandis que celui entre les niveaux d'expression de (A') et (B) est proche de 1.

Le procédé sera décrit plus précisément au moyen des exemples ci-dessous.

### Exemples

### Exemple 1

Afin d'identifier des marqueurs biologiques caractérisant la peau des enfants, les présents inventeurs ont entrepris une étude d'analyse génomique en fonction de l'âge des enfants.

### Matériels et méthodes

Des échantillons de peau ont été prélevés (prélèvement préputial ou plastie mammaire, selon le sexe du donneur) sur des donneurs âgés de 1 mois, 3 mois, 3 ans, 6 ans et 11 ans, ainsi que sur des adultes.

Des modèles de peau reconstruite ont été générés à partir de ces échantillons. Des épidermes reconstruits ont ainsi été réalisés avec les différents donneurs sélectionnés par la technique de Poumay et al. (Arch Dermatol Res, 296(5) : 203-211, 2004). Après 2 jours de culture en immersion, les épidermes reconstruits ont été cultivés à l'interface air/liquide pendant 5 ou 9 jours. Pendant la durée de la culture, le milieu a été renouvelé tous les 2 ou 3 jours.

À la fin de l'incubation, les épidermes ont été rincés et pour moitié congelés et pour moitié fixés dans une solution de formaldéhyde. Des coupes transversales ont été réalisées dans les tissus fixés au microtome, afin de vérifier la structure des épidermes.

Parallèlement à ces épidermes reconstruits, des cultures monocouches de kératinocytes provenant des différents échantillons ont été réalisées. Les différents kératinocytes ont été ensemencés en milieu de culture en plaque 12 puits. Après 48 heures d'incubation (jour du passage air liquide pour les épidermes reconstruits) les tapis cellulaires ont été lysés en TriPure Isolation Reagent® (Roche Applied Science) et congelés à -80°C.

À la fin de l'incubation, les épidermes ou les tapis cellulaires ont été lysés en TriPure Isolation Reagent® et les ARN totaux ont été extraits selon le protocole préconisé par le fournisseur. La quantité et la qualité des ARN ont été évaluées à l'aide du Bioanalyzer (Agilent Technologies). Chaque échantillon d'ARN a été aliquoté, une partie de l'échantillon étant utilisée pour une hybridation sur micropuces, le reste étant congelé avant d'être utilisé pour une expérience de RT-PCR quantitative.

Les ARN ont été amplifiés et marqués à l'aide du GeneChip 3'IVT Express Kit (Affimetrix) en suivant le protocole recommandé par le fournisseur. Ils ont ensuite été hybridés sur la puce Affymetrix® U219 dans la station d'hybridation GeneAtlas™ fluidics station Affymetrix® pendant 16 heures à 45°C. Cette étape a été réalisée à l'aide du kit « GeneAtlas™ hybridization, wash and stain kit for 3' IVT arrays » (Affymetrix®). La puce U219 a été analysée à l'aide du scanner GeneAtlasTM Imaging station.

Les données obtenues par hybridation sur puce ont été traitées et normalisées à l'aide des logiciels fournis par Affymetrix (GCOS Affymetrix®, Expression Console Affymetrix®).

Les résultats obtenus par hybridation sur puce ont été confirmés par analyse par RT-PCR quantitative (RT-qPCR). Pour cela, les ARN préparés sont d'abord rétro-transcrits en ADNc an présence d'oligo(dT) à l'aide de l'enzyme Superscript II (Gibco).

### Résultats

L'analyse histologique a montré que tous les épidermes reconstruits avaient la morphologie attendue à J5 et J12, quel que soit le donneur, permettant de valider les résultats d'analyse génique. En particulier, on retrouvait bien la couche basale, la couche épineuse, la couche granuleuse et la couche cornée dans chaque épiderme. De plus, comme attendu, l'épaisseur de ceux-ci était plus importante à J12 qu'à J5.

L'analyse génomique a permis d'identifier plusieurs marqueurs dont l'expression varie en fonction de l'âge du donneur. Ces résultats ont été confirmés par RT-PCR quantitative. Les marqueurs ont ensuite été regroupés par fonction biologique, à l'aide du logiciel Pathway Studio® d'Ariadne Genomics.

On a ainsi pu identifier un groupe de gènes de la barrière cutanée, comprenant les gènes CLDN1, IVL, KRT1. L'expression de ces gènes de la fonction barrière épidermique augmente avec l'âge dans les épidermes reconstruits (Figure 1A) ; en revanche, ces gènes sont moins exprimés chez les plus jeunes. Ces résultats ont été confirmés par l'analyse en RT-PCR quantitative dans les kératinocytes (Figure 1B). Les résultats obtenus avec les épidermes reconstruits et les kératinocytes sont similaires en termes de profil d'expression, ce qui valide l'approche entreprise. Il est d'ailleurs intéressant de noter que ces résultats, tout comme ceux de Fluhr et al. (Exp Dermatol., 19(6) : 483-492, 2010), illustrent la maturation avec l'âge de la fonction barrière de la peau.

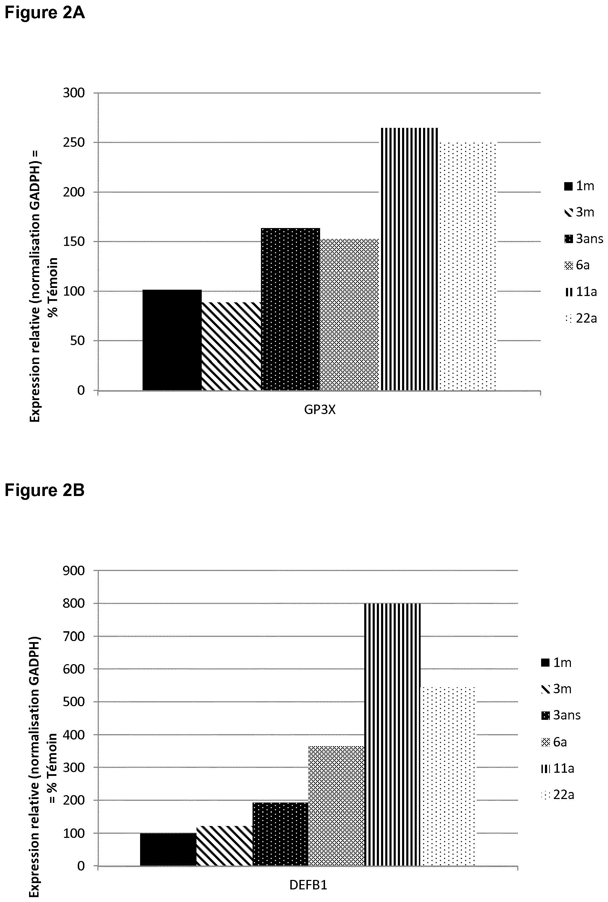

De même, l'expression des gènes de réponse au stress (GPX3) et de défense innée (DEFB1) augmente avec l'âge (Figures 2A et 2B, respectivement).

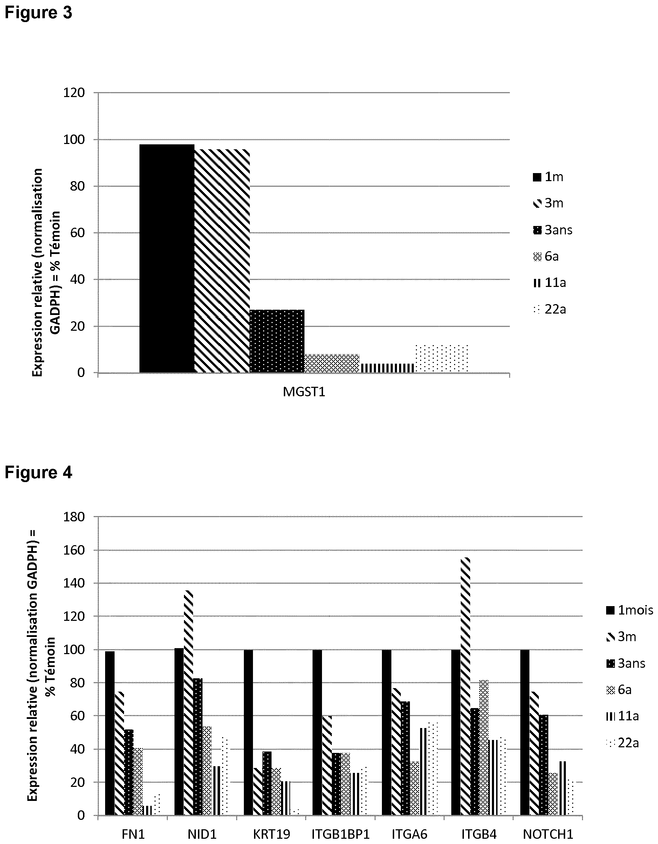

En revanche, l'expression du gène MGST1 diminue avec l'âge (Figure 3). Ce gène est exprimé fortement chez les enfants de 1 et 3 mois, mais sa transcription décroît ensuite d'un facteur au moins égal à 5.

Enfin, l'expression des gènes des cellules souches, FN1, NOTCH1, NID1, KRT19, ITGBP1, ITGA6 et ITGB4, est importante après la naissance et diminue avec l'âge (Figure 4).

### Exemple 2

La tolérance de produits à base de sucres en C7 d'avocat (perséose d'avocat, voir par exemple WO 2005/115421, WO 2008/025847 ou WO 2011/073281) a été évaluée sur des épidermes reconstruits provenant de donneurs âgés de 1 mois.

### Matériel et méthodes

Des épidermes ont été reconstruits comme indiqué dans l'exemple 1 avec des kératinocytes provenant de donneurs de 1 mois. Les produits, un lait de toilette et une lotion hydratante contenant tous deux du perséose d'avocat, ont été appliqués sur ces épidermes reconstruits durant 16 heures. Un contrôle a été réalisé en appliquant du SDS 0,4 % sur des échantillons d'épidermes reconstruits témoins.

La protéine IL-8 a été dosée dans les surnageants de culture par un test Elisa. La viabilité cellulaire a été évaluée par le test MTT. Enfin, une analyse histologique a été réalisée sur des tissus inclus en paraffine par coloration hématoxyline et éosine.

### Résultats

Les résultats rapportés dans le tableau 1 montrent que la viabilité cellulaire n'est pas affectée par l'application du lait de toilette et de la lotion hydratante contenant du perséose d'avocat. En comparaison, la viabilité des cellules de l'épiderme traité au SDS est très fortement diminuée, puisque seules 2 % des cellules sont encore vivantes après le traitement.

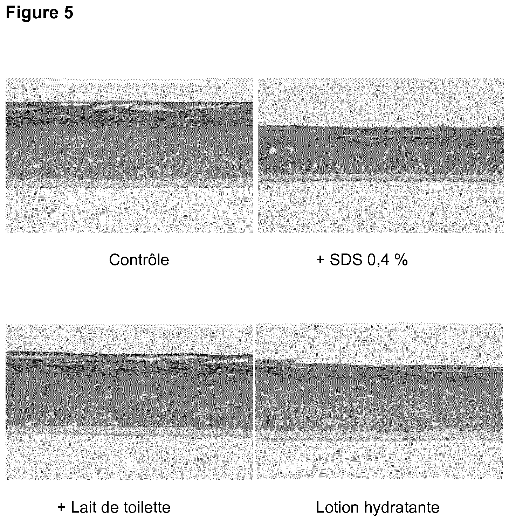

Par ailleurs, l'épaisseur des couches de cellules vivantes est maintenue à un niveau similaire à celui du témoin dans les échantillons traités avec les produits contenant du perséose d'avocat. En comparaison, l'analyse histologique montre que les couches de cellules vivantes dans l'épiderme traité au SDS ne correspondent plus qu'à 50-60 % de l'épaisseur observée chez le contrôle (Figure 5).

Ces résultats de biologie cellulaire sont confirmés par l'analyse de l'activation de la cytokine IL-8 en réponse aux différents traitements. Alors que le SDS entraîne une forte augmentation de l'expression de cette protéine, aucun effet n'est observé quand les épidermes reconstruits sont traités avec le lait de toilette ou la lotion hydratante contenant du perséose d'avocat.

L'ensemble de ces résultats montre donc que les deux produits contenant du perséose d'avocat ne sont pas toxiques et sont bien tolérés.

**Tableau 1**

| | Contrôle | + SDS 0,4% | + Lait de toilette | + Lotion hydratante |
|---|---|---|---|---|
| Viabilité | 100% | 2% | 84% | 93% |
| Molécule inflammatoire IL8 | 100% | 602% | 99% | 89% |
| Epaisseur couches vivantes | 100% | 58% | 88% | 87% |

## Revendications

1. Procédé d'identification d'au moins un marqueur biologique caractérisant une peau d'enfant, ledit procédé comprenant les étapes suivantes :
a) mesurer le niveau d'expression d'un marqueur biologique candidat dans au moins deux cultures de peau reconstruite (A) et (A'), lesdites cultures provenant d'un donneur choisi dans le groupe constitué des nouveau-nés, dont l'âge est compris entre 0 et 1 mois, et des nourrissons, dont l'âge est compris entre 1 mois et 2 ans ; lesdites cultures (A) et (A') appartenant à deux classes d'âge différentes,
b) mesurer le niveau d'expression dudit marqueur biologique candidat dans au moins une culture de peau reconstruite témoin (B),
c) calculer le rapport entre le niveau d'expression de l'étape a) et le niveau d'expression de l'étape b), et
d) déterminer que le marqueur candidat est un marqueur biologique caractérisant une peau d'enfant, si
• la culture témoin (B) provient d'un adulte et
• le rapport de l'étape c) est différent de 1.

2. Procédé selon la revendication 1, **caractérisé en ce que** ledit marqueur biologique est un marqueur génique, un marqueur protéique, un marqueur lipidique ou un marqueur métabolique.

3. Procédé selon l'une quelconque des revendications 1 ou 2, **caractérisé en ce que** ledit marqueur biologique candidat est un marqueur génique sélectionné dans le groupe comprenant les marqueurs du métabolisme cellulaire, les marqueurs de la réponse au stress, les marqueurs de l'inflammation, les marqueurs de l'immunité, les marqueurs d'apoptose, les marqueurs de croissance/prolifération et du cycle cellulaire, les marqueurs de la signalisation cellulaire, les marqueurs de migration et de différenciation, les marqueurs de de la barrière épidermique, les marqueurs d'adhésion et les marqueurs des cellules souches pluripotentes de la peau.

4. Procédé selon l'une quelconque des revendications 2 et 3 , **caractérisé en ce que** le niveau d'expression dudit marqueur génique est déterminé par une méthode sélectionnée parmi le Northern blot, le Southern blot, la PCR, la RT-PCR, la RT-PCR quantitative, le SAGE et ses dérivés, les puces d'acides nucléiques, notamment les puces à ADNc, les puces à oligonucléotides et les puces à ARNm, les puces à tissu et le RNA-Seq.

5. Procédé selon l'une quelconque des revendications 1 et 2, **caractérisé en ce que** ledit marqueur biologique candidat est un marqueur protéique sélectionné dans le groupe comprenant les marqueurs du métabolisme cellulaire, les marqueurs de la réponse au stress, les marqueurs de l'inflammation et de l'immunité, les marqueurs d'apoptose, les marqueurs de croissance/prolifération et du cycle cellulaire, les marqueurs de la signalisation cellulaire, les marqueurs de migration et de différenciation, les marqueurs de de la barrière épidermique, les marqueurs d'adhésion et les marqueurs des cellules souches pluripotentes de la peau.

6. Procédé selon l'une quelconque des revendications 2 et 5, **caractérisé en ce que** le niveau d'expression dudit marqueur protéique est déterminé par une méthode sélectionnée parmi, l'immunohistologie, l'immunoprécipitation, le western blot, le dot blot, l'ELISA ou l'ELISPOT, les puces à protéines, les puces à anticorps, ou les puces à tissu couplées à l'immunohistochimie, les techniques de FRET ou de BRET, les méthodes de microscopie ou d'histochimie, dont notamment les méthodes de microscopie confocale et de microscopie électronique, les méthodes basées sur l'utilisation d'une ou plusieurs longueurs d'onde d'excitation et d'une méthode optique adaptée, comme une méthode électrochimique (les techniques voltammétrie et d'ampérometrie), le microscope à force atomique, et les méthodes de radiofréquence, comme la spectroscopie résonance multipolaire, confocale et non-confocale, détection de fluorescence, luminescence, chemiluminescence, absorbance, réflectance, transmittance, and biréfringence ou index de réfraction (par exemple, par résonance des plasmons de surface, ou « surface plasmon resonance » en anglais, par ellipsometry, par méthode de miroir résonnant, tec.), cytométrie de flux, imagerie par résonance radioisotopique ou magnétique, analyse par électrophorèse en gel de polyacrylamide (SDS-PAGE); par spectrophotométrie HPLC-Mass, par chromatographie liquide/spectrophotométrie de masse/spectrométrie de masse (LC-MS/MS).

7. Procédé selon l'une quelconque des revendications 1 et 2, **caractérisé en ce que** ledit marqueur candidat est un marqueur lipidique de la couche cornée, préférentiellement choisi parmi les céramides, le cholestérol et les acides gras libres, un marqueur lipidique du sébum ou un marqueur du film hydrolipidique.

8. Procédé selon la revendication 7 **caractérisé en ce que** ledit marqueur lipidique est un céramide choisi dans le groupe constitué par les céramides CER 1 à 9.

9. Procédé selon l'une quelconque des revendications 2, 7 et 8, **caractérisé en ce que** le niveau d'expression dudit marqueur lipidique est déterminé par une méthode sélectionnée parmi la chromatographie liquide en phase gazeuse (HPLC), notamment la chromatographie liquide en phase gazeuse couplée à un détecteur évaporatif à diffraction de la lumière (HPLC-ESD) ; la chromatographie en couche mince (TLC) ; la résonance magnétique nucléaire (NMR) ; la microspectroscopie confocale in vivo de Raman ; la spectrométrie de masse, la chromatographie en phase gazeuse couplée à la spectrométrie de masse (GC-MS) ; et la chromatographie en phase liquide ultra-performante (UPLC).

10. Procédé selon l'une quelconque des revendications 1 et 2, **caractérisé en ce que** ledit marqueur candidat est un marqueur métabolique choisi parmi marqueurs de la construction de l'épiderme et de la fonction barrière de la peau, notamment les facteurs naturels d'hydratation, aussi appelé NMF (Natural Moisturizing Factor) ; les hormones de la peau ; les activités enzymatiques antioxydantes ; les activités enzymatiques liées à l'élimination des composés toxiques exogènes, notamment les enzymes de phase I et les enzymes de phase II ; les marqueurs liés à la pigmentation de la peau, notamment la mélanine et les activités enzymatiques régulant la synthèse de la mélanine ; les marqueurs liés aux adipocytes, notamment le rapport entre les adipocytes blancs et les adipocytes bruns, les adipocytokines et les activités enzymatiques liées à la lipolyse.

11. Procédé d'évaluation de la tolérance d'un agent actif, d'une composition dermo-cosmétique ou d'une formulation cosmétique, comprenant les étapes suivantes :
a) obtenir au moins une culture de peau reconstruite (A) provenant d'un donneur choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans ;
b) mettre en contact un agent actif, d'une composition dermo-cosmétique ou d'une formulation cosmétique avec la culture (A) ;
c) mesurer le niveau d'expression d'au moins un marqueur biologique caractérisant une peau d'enfant;
d) mesurer le niveau d'expression dudit marqueur biologique dans une culture témoin ;
e) calculer le rapport entre le niveau d'expression de l'étape c) et le niveau d'expression d'étape d), et
f) déterminer que ledit agent actif, la composition dermo-cosmétique ou la formulation cosmétique est bien toléré par la culture de peau reconstruite si ledit agent actif, ladite composition dermo-cosmétique ou ladite formulation cosmétique n'induit pas l'expression dudit marqueur biologique ;
dans lequel ledit marqueur biologique est un marqueur de l'inflammation choisi parmi IL-1 et IL-8.

12. Procédé selon la revendication 11, **caractérisé en ce que** l'échantillon témoin de l'étape d) est la culture qui n'a pas été mise en contact avec l'agent actif, la composition dermo-cosmétique ou la formulation cosmétique.

13. Procédé selon l'une quelconque des revendications 11 ou 12, **caractérisé en ce que** ledit procédé comprend une étape supplémentaire de détermination de la viabilité cellulaire dans la culture (A) traitée avec l'agent actif, la composition dermo-cosmétique ou la formulation cosmétique, de détermination de la viabilité cellulaire dans la culture témoin et de comparaison entre les deux.

14. Procédé selon l'une quelconque des revendications 11 à 13, **caractérisé en ce que** les cellules de ladite culture proviennent d'un explant de tissu cutané ou de cellules souches différenciées en cellules cutanées.

15. Procédé selon l'une quelconque des revendications 11 à 14, **caractérisé en ce que** la culture comprend au moins des fibroblastes ou des kératinocytes.

16. Procédé selon l'une quelconque des revendications 11 à 15, **caractérisé en ce que** la peau reconstruite est sélectionnée dans le groupe comprenant les modèles de derme, les modèles d'épiderme, les modèles d'hypoderme, les modèles de peau comprenant un derme et un épiderme, et les modèles de peau comprenant un derme, un épiderme et un hypoderme.

## Patentansprüche

1. Verfahren zur Identifizierung mindestens eines biologischen Markers, der eine Kinderhaut kennzeichnet, wobei das Verfahren die folgenden Schritte umfasst:
a) Messen des Expressionsniveaus eines biologischen Markerkandidaten in mindestens zwei rekonstruierten Hautkulturen (A) und (A'), wobei die Kulturen von einem Spender stammen, der aus der Gruppe ausgewählt ist, die von den Neugeborenen, deren Alter zwischen 0 und 1 Monat liegt, und den Säuglingen, deren Alter zwischen 1 Monat und 2 Jahren liegt, gebildet ist; wobei die Kulturen (A) und (A') zu zwei verschiedenen Altersklassen gehören,
b) Messen des Expressionsniveaus des biologischen Markerkandidaten in mindestens einer rekonstruierten Vergleichs-Hautkultur (B),
c) Berechnen des Verhältnisses zwischen dem Expressionsniveau von Schritt a) und dem Expressionsniveau von Schritt b), und
d) Bestimmen, dass der Markerkandidat ein biologischer Marker ist, der eine Kinderhaut kennzeichnet, wenn
• die Vergleichskultur (B) von einem Erwachsenen stammt, und
• sich das Verhältnis von Schritt c) von 1 unterscheidet.

2. Verfahren nach Anspruch 1, **dadurch gekennzeichnet, dass** der biologische Marker ein genetischer Marker, ein Proteinmarker, ein Lipidmarker oder ein Stoffwechselmarker ist.

3. Verfahren nach einem der Ansprüche 1 oder 2, **dadurch gekennzeichnet, dass** der biologische Markerkandidat ein genetischer Marker ist, der aus der Gruppe ausgewählt ist, die die Marker des Zellstoffwechsels, die Marker der Stressantwort, die Entzündungsmarker, die Immunitätsmarker, die Apoptosemarker, die Wachstums-/Verbreitungsmarker und des zellulären Zyklus, die Marker der Zellsignalisierung, die Migrations- und Differenzierungsmarker, die Marker der Epidermisbarriere, die Adhäsionsmarker und die Marker der pluripotenten Stammzellen der Haut umfasst.

4. Verfahren nach einem der Ansprüche 2 und 3, **dadurch gekennzeichnet, dass** das Expressionsniveau des genetischen Markers durch eine Methode bestimmt wird, die aus Northern Blot, Southern Blot, PCR, RT-PCR, der quantitativen RT-PCR, SAGE und deren Ableitungen, den Nukleinsäure-Chips, insbesondere den cDNA-Chips, den Oligonukleotid-Chips und den mRNA-Chips, den Gewebechips und der RNA-Seq ausgewählt ist.

5. Verfahren nach einem der Ansprüche 1 und 2, **dadurch gekennzeichnet, dass** der biologische Markerkandidat ein Proteinmarker ist, der aus der Gruppe ausgewählt ist, die die Marker des Zellstoffwechsels, die Marker der Stressantwort, die Entzündungs- und Immunitätsmarker, die Apoptosemarker, die Wachstums-/Verbreitungsmarker und des zellulären Zyklus, die Marker der Zellsignalisierung, die Migrations- und Differenzierungsmarker, die Marker der Epidermisbarriere, die Adhäsionsmarker und die Marker der pluripotenten Stammzellen der Haut umfasst.

6. Verfahren nach einem der Ansprüche 2 und 5, **dadurch gekennzeichnet, dass** das Expressionsniveau des Proteinmarkers durch eine Methode bestimmt wird, die aus der Immunhistologie, der Immunpräzipitation, Western Blot, Dot Blot, ELISA oder ELISPOT, den Protein-Chips, den Antikörper-Chips oder den Gewebechips, gekoppelt an die Immunhistochimie, den Techniken FRET oder BRET, den Mikroskopie- oder Histochemiemethoden, darunter vor allem den Methoden der Konfokalmikroskopie und der elektronischen Mikroskopie, den Methoden, die auf der Verwendung einer oder mehrerer Erregungswellenlängen und einer entsprechenden optischen Methode basieren, wie eine elektrochemische Methode (die Voltammetrie- und Amperometrietechniken), dem Rasterkraftmikroskop und den Radiofrequenzmethoden wie die multipolare, konfokale und nicht-konfokale Resonanzspektroskopie, Fluoreszenz-, Lumineszenz-, Chemilumineszenz-, Absorbanz-, Reflektanz-, Transmittanzdetektion und Doppelbrechung oder Refraktionsindex (beispielsweise durch Oberflächenplasmonresonanz oder "surface plasmon resonance" in Englisch, durch Ellipsometrie, durch Resonanzspiegelmethode, usw.), Durchflusszytometrie, Bildgebung durch Radioisotop- oder Magnetresonanz, Analyse durch Polyacrylamidgelelektrophorese (SDS-PAGE), durch HPLC-Mass-Spektrophotometrie, durch Flüssig-/Massenspektrophotometrie-/Massenspektrometrie-Chromatographie (LC-MS/MS) ausgewählt ist.

7. Verfahren nach einem der Ansprüche 1 und 2, **dadurch gekennzeichnet, dass** der Markerkandidat ein Lipidmarker der Hornschicht, vorzugsweise ausgewählt aus den Ceramiden, dem Cholesterol und den freien Fettsäuren, ein Lipidmarker des Sebums oder ein Marker des Hydrolipidfilms ist.

8. Verfahren nach Anspruch 7, **dadurch gekennzeichnet, dass** der Lipidmarker ein Ceramid ist, ausgewählt aus der Gruppe, die von den Ceramiden CER 1 bis 9 gebildet ist.

9. Verfahren nach einem der Ansprüche 2, 7 und 8, **dadurch gekennzeichnet, dass** das Expressionsniveau des Lipidmarkers durch eine Methode bestimmt wird, die aus der High-Performance-Flüssigkeitschromatographie (HPLC), insbesondere der High-Performance-Flüssigkeitschromatographie, gekoppelt an einen Lichtstreudetektor (HPLC-ESD); der Dünnschichtchromatographie (TLC); der Kernspinresonanz (NMR); der konfokalen in vivo-Raman-Mikrospektroskopie; der Massenspektrometrie, der Gaschromatographie, gekoppelt an die Massenspektrometrie (GC-MS) und der Ultra-Performance-Flüssigkeitschromatographie (UPLC) ausgewählt ist.

10. Verfahren nach einem der Ansprüche 1 und 2, **dadurch gekennzeichnet, dass** der Markerkandidat ein Stoffwechselmarker ist, der aus den Markern der Konstruktion der Epidermis und der Barrierefunktion der Haut, insbesondere den natürlichen Feuchthaltefaktoren, auch als NMF (Natural Moisturizing Factor) bezeichnet; den Hormonen der Haut; den antioxidativen enzymatischen Aktivitäten; den enzymatischen Aktivitäten, die mit der Entfernung exogener toxischer Verbindungen verbunden sind, insbesondere den Enzymen von Phase I und den Enzymen von Phase II; den Markern, die mit der Pigmentierung der Haut verbunden sind, insbesondere dem Melanin und den enzymatischen Aktivitäten, die die Melaninsynthese steuern; den Markern, die mit den Adipozyten verbunden sind, insbesondere dem Verhältnis zwischen den weißen Adipozyten und den braunen Adipozyten, den Adipozytokinen und den enzymatischen Aktivitäten, die mit der Lipolyse verbunden sind, ausgewählt ist.

11. Verfahren zur Evaluierung der Verträglichkeit eines Wirkstoffs, einer dermokosmetischen Zusammensetzung oder einer kosmetischen Formulierung, umfassend die folgenden Schritte:
a) Erhalten mindestens einer rekonstruierten Hautkultur (A) von einem Spender, der aus der Gruppe ausgewählt ist, die von den Neugeborenen, deren Alter zwischen 0 und 1 Monat liegt, den Säuglingen, deren Alter zwischen 1 Monat und 2 Jahren liegt, und den Kindern, deren Alter zwischen 2 und 16 Jahren liegt, gebildet ist,
b) Inkontaktversetzen eines Wirkstoffs, einer dermokosmetischen Zusammensetzung oder einer kosmetischen Formulierung mit der Kultur (A);
c) Messen des Expressionsniveaus mindestens eines biologischen Markers, der eine Kinderhaut kennzeichnet;
d) Messen des Expressionsniveaus des biologischen Markers in einer Vergleichskultur;
e) Berechnen des Verhältnisses zwischen dem Expressionsniveau von Schritt c) und dem Expressionsniveau von Schritt d), und
f) Bestimmen, dass der Wirkstoff, die dermokosmetische Zusammensetzung oder die kosmetische Formulierung von der rekonstruierten Hautkultur gut vertragen wird, wenn der Wirkstoff, die dermokosmetische Zusammensetzung oder die kosmetische Formulierung keine Expression des biologischen Markers induziert;
wobei der biologische Marker ein Entzündungsmarker ist, ausgewählt aus IL-1 und IL-8.

12. Verfahren nach Anspruch 11, **dadurch gekennzeichnet, dass** die Vergleichsprobe von Schritt d) die Kultur ist, die nicht mit dem Wirkstoff, der dermokosmetischen Zusammensetzung oder der kosmetischen Formulierung in Kontakt versetzt wird.

13. Verfahren nach einem der Ansprüche 11 oder 12, **dadurch gekennzeichnet, dass** das Verfahren einen zusätzlichen Schritt des Bestimmens der zellulären Lebensfähigkeit in der Kultur (A), behandelt mit dem Wirkstoff, der dermokosmetischen Zusammensetzung oder der kosmetischen Formulierung, des Bestimmens der zellulären Lebensfähigkeit in der Vergleichskultur und des Vergleichs zwischen den beiden umfasst.

14. Verfahren nach einem der Ansprüche 11 bis 13, **dadurch gekennzeichnet, dass** die Zellen der Kultur von einem Hautgewebsexplantat oder von in Hautzellen differenzierten Stammzellen stammen.

15. Verfahren nach einem der Ansprüche 11 bis 14, **dadurch gekennzeichnet, dass** die Kultur mindestens Fibroblasten oder Keratinozyten umfasst.

16. Verfahren nach einem der Ansprüche 11 bis 15, **dadurch gekennzeichnet, dass** die rekonstruierte Haut aus der Gruppe ausgewählt ist, die die Dermismodelle, die Epidermismodelle, die Hypodermismodelle, die Hautmodelle, die eine Dermis und eine Epidermis und die Hautmodelle, die eine Dermis, eine Epidermis und eine Hypodermis umfassen, umfasst.

## Claims

1. A process for identifying at least one biological marker characterizing children's skin, said process comprising the following steps:
a) measuring the level of expression of a candidate biological marker in at least two reconstructed skin cultures (A) and (A'), said cultures coming from a donor selected from the group consisting of newborns, whose age is between 0 and 1 month, and infants, whose age is between 1 month and 2 years; said cultures (A) and (A') belonging to two different age classes,
b) measuring the level of expression of said candidate biological marker in at least one control reconstructed skin culture (B),
c) calculating the ratio between the level of expression of step a) and the level of expression of step b), and
d) determining that the candidate marker is a biological marker characterizing children's skin, if
• the control culture (B) comes from an adult and
• the ratio in step c) is different from 1.

2. The process according to claim 1, **characterized in that** said biological marker is a genetic marker, a protein marker, a lipidic marker or a metabolic marker.

3. The process according to any one of claim 1 or claim 2, **characterized in that** said candidate biological marker is a genetic marker selected from the group comprising cell metabolism markers, stress response markers, inflammation markers, immunity markers, apoptosis markers, cell growth/proliferation and cell-cycle markers, cell signaling markers, migration and differentiation markers, epidermal barrier markers, adhesion markers and pluripotent skin stem cell markers.

4. The process according to any one of claims 2 and 3, **characterized in that** the level of expression of said genetic marker is determined by a method selected from Northern blot, Southern blot, PCR, RT-PCR, quantitative RT-PCR, SAGE and derivatives thereof, nucleic acid chips, in particular cDNA chips, oligonucleotide chips and mRNA chips, tissue chips and RNA-Seq.

5. The process according to any one of claims 1 and 2, **characterized in that** said candidate biological marker is a protein marker selected from the group comprising cell metabolism markers, stress response markers, inflammation and immunity markers, apoptosis markers, cell growth/proliferation and cell-cycle markers, cell signaling markers, migration and differentiation markers, epidermal barrier markers, adhesion markers and pluripotent skin stem cell markers.

6. The process according to any one of claims 2 and 5, **characterized in that** the level of expression of said protein marker is determined by a method selected from immunohistology, immunoprecipitation, Western blot, dot blot, ELISA or ELISPOT, protein chips, antibody chips, or tissue chips coupled with immunohistochemistry, FRET or BRET techniques, microscopy or histochemistry methods, including in particular confocal and electron microscopy methods, methods based on the use of one or more excitation wavelengths and a suitable optical method, such as an electrochemical method (voltammetric and amperometric techniques), atomic force microscopy, and radiofrequency methods, such as multipolar resonance spectroscopy, confocal and non-confocal, detection of fluorescence, luminescence, chemiluminescence, absorbance, reflectance, transmittance, and birefringence or refractive index (for example, by surface plasmon resonance, by ellipsometry, by resonant mirror method, etc.), flow cytometry, radioisotopic or magnetic resonance imaging, polyacrylamide gel electrophoresis analysis (SDS-PAGE); HPLC-mass spectrophotometry, liquid chromatography/mass spectrophotometry/mass spectrometry (LC-MS/MS) .

7. The process according to any one of claims 1 and 2, **characterized in that** said candidate marker is a corneal layer lipidic marker, preferentially selected from ceramides, cholesterol and free fatty acids, a sebum lipidic marker or a hydrolipidic film marker.

8. The process as claimed in claim 7 **characterized in that** said lipidic marker is a ceramide selected from the group consisting of ceramides CER 1 to 9.

9. The process according to any one of claims 2, 7 and 8, **characterized in that** the level of expression of said lipidic marker is determined by a method selected from gas phase liquid chromatography (HPLC), in particular gas phase liquid chromatography coupled with an evaporative light scattering detector (HPLC-ESD); thin-layer chromatography (TLC); nuclear magnetic resonance (NMR); *in vivo* confocal Raman microspectroscopy; mass spectrometry, gas chromatography coupled with mass spectrometry (GC-MS); and ultra-performance liquid chromatography (UPLC).

10. The process according to any one of claims 1 and 2, **characterized in that** said candidate marker is a metabolic marker selected from markers of epidermal construction and skin barrier function, in particular natural moisturizing factors (NMF); skin hormones; antioxidant enzyme activities; enzyme activities related to the elimination of exogenous toxic compounds, in particular phase I and phase II enzymes; markers related to skin pigmentation, in particular melanin and enzyme activities regulating melanin synthesis; markers related to adipocytes, in particular the ratio of white to brown adipocytes, adipocytokines and enzyme activities related to lipolysis.

11. A process for evaluating the tolerance of an active agent, a dermocosmetic composition or a cosmetic formulation, comprising the following steps:
a) obtaining at least one reconstructed skin culture (A) from a donor selected from the group consisting of newborns, whose age is between 0 and 1 month, infants, whose age is between 1 month and 2 years, and children whose age is between 2 years and 16 years;
b) contacting an active agent, a dermocosmetic composition or a cosmetic formulation with culture (A);
c) measuring the level of expression of at least one biological marker characterizing children's skin;
d) measuring the level of expression of said biological marker in a control culture;
e) calculating the ratio between the level of expression of step c) and the level of expression of step d), and
f) determining whether said active agent, the dermocosmetic composition or the cosmetic formulation is well tolerated by the reconstructed skin culture if said active agent, said dermocosmetic composition or said cosmetic formulation does not induce the expression of said biological marker;
wherein said biological marker is an inflammation marker selected from IL-1 and IL-8.

12. The process according to claim 11, **characterized in that** the control sample of step d) is the culture which has not been contacted with the active agent, the dermocosmetic composition or the cosmetic formulation.

13. The process according to any one of claim 11 or 12, **characterized in that** said process comprises an additional step of determining the cell viability in the culture (A) treated with the active agent, the dermocosmetic composition or the cosmetic formulation, determining the cell viability in the control culture and comparing the two.

14. The process according to any one of claims 11 to 13, **characterized in that** the cells of said culture are derived from an explant of skin tissue or from stem cells differentiated into skin cells.

15. The process according to any one of claims 11 to 14, **characterized in that** the culture comprises at least fibroblasts or keratinocytes.

16. The process as claimed in any one of claims 11 to 15, **characterized in that** the reconstructed skin is selected from the group consisting of dermal models, epidermal models, hypodermal models, skin models comprising a dermis and an epidermis, and skin models comprising a dermis, an epidermis and a hypodermis.
